# EUROPEAN PATENT SPECIFICATION

(11) **EP 3 818 361 B1**
(45) Date of publication and mention of the grant of the patent: **24.01.2024**
(21) Application number: 19831543.4
(22) Date of filing: 02.07.2019
(51) Int. Cl.: G01N 27/00, G01N 27/02, G01N 33/50, G01N 33/483

(54) **TOPOGRAPHICALLY GUIDED CELL MIGRATION DEVICES AND METHODS**
VORRICHTUNGEN UND VERFAHREN ZUR TOPOGRAFISCH GEFÜHRTEN ZELLMIGRATION
DISPOSITIFS ET PROCÉDÉS DE MIGRATION CELLULAIRE À GUIDAGE TOPOGRAPHIQUE

(30) Priority: 03.07.2018 US 201862693473 P
(43) Date of publication of application: 12.05.2021
(73) Proprietor: Curi Bio, Inc., Seattle, Washington 98121-1023 (US); University of Washington, Seattle, Washington 98105-4721 (US)
(72) Inventor: KIM, Deok-Ho, Seattle, WA 98105-4721 (US); GRAY, Kevin, Seattle, Washington 98121-1023 (US); CHOI, Jongseob, Seattle, WA 98105-4721 (US)
(74) Representative: Grünecker Patent- und Rechtsanwälte PartG mbB
(86) International application number: PCT/US2019/040417
(87) International publication number: WO 2020/010154

(56) References cited:
- WO-A1-2016/129894
- US-A1- 2004 142 411
- US-A1- 2005 112 544
- US-A1- 2005 267 528
- US-A1- 2008 257 735
- US-A1- 2009 036 988
- US-A1- 2010 279 320
- US-A1- 2012 029 557
- US-A1- 2013 171 682
- US-A1- 2014 243 243
- TIEN ANH NGUYEN ET AL: "Microfluidic Chip with Integrated Electrical Cell-Impedance Sensing for Monitoring Single Cancer Cell Migration in Three-Dimensional Matrixes", ANALYTICAL CHEMISTRY, vol. 85, no. 22, 8 November 2013 (2013-11-08), pages 11068-11076, XP055652686, US ISSN: 0003-2700, DOI: 10.1021/ac402761s
- HAN et al.: "Unidirectional migration of single smooth muscle cells under the synergetic effects of gradient swelling cue and parallel groove patterns", Colloids and Surfaces B: Biointerfaces, vol. 111, November 2013 (2013-11), pages 1-6, XP029056475, DOI: 10.1016/j.colsurfb.2013.05.011

## Description

### BACKGROUND

Cancer is a leading cause of death worldwide, and is among the most notorious of human diseases, in large part due to its uncontrollable metastatic characteristics. The survival rate of late-stage patients with tumor metastasis is low even after intensive treatment. Metastatic tumors, rather than primary tumors, cause over 90% of cancer-associated deaths. Even patients with localized diseases also have a high risk of developing tumor metastasis and recurrence. However, currently available cancer therapies mainly aim to achieve cytotoxicity. These cytoreductive therapies alone are not enough to mitigate cancer metastasis and progression. The fact that no effective anti-metastasis drug is available for clinical cancer patients accentuates the urgent need to identify drug targets that can inhibit migration of the cancer cells.

Investigations on cancer migration patterns reveal distinct migration strategies used by different kinds of cancer cells. For example, epithelial cancer cells usually migrate collectively in a cell cluster where the cells are connected in junctions. Migrating together, these cells form a cluster composed of polyclonal cells which exhibits higher tolerances of microenvironmental stress in distant metastasis sites. Moreover, the cancer cells of a leading group in such collective migration adapt to and remodel the underlying extracellular matrix by creating sheets of aligned nanoscale collagen fibrils, thereby effectively forming a "migration highway" that guides the migrating cancer cells through the stroma to produce metastases at the distant sites. Although isolated single cancer cells, rather than tumor clusters, are often observed at a tumor invasion border when viewed on microscope slides prepared for clinical analysis or diagnosis, 3D histopathological image analysis shows that these single cancer cells observed in 2D sections are, in fact, connected to nearby cancer cell clusters, demonstrating sound clinical evidence that epithelial cancer cells migrate collectively.

Hence, there is a critical need for an *in vitro* assay tool which can recapitulate the migratory behaviors of the cells *in vivo.* Such an assay tool should provide a means for directing cells to migrate collectively along aligned nanoscale features reminiscent of the metastatic microenvironment *in vivo.* However, conventional *in vitro* assay models for assessing cell migration behaviors (such as scratch, barrier, and electric fence assays) do not provide any relevant tumor microenvironment features. In a widely used Boyden chamber assay, Matrigel could be coated to recapitulate tumor stromal components; however, such transmembrane Boyden chamber restricts the cells to migrate only in a single-cell pattern. In 3D models, multicellular spheroids or organoid culture could be planted into 3D collagen or extracellular matrix (ECM) that mimics the relevant tumor microenvironment; however, it is difficult and time-consuming to perform an image analysis, or obtain quantified experimental data, using such 3D models. What is needed, therefore, is a device, system and method for cell assay platforms that can recapitulate *in vitro* the metastatic microenvironment of tumor cells while allowing a reliable, easily quantifiable, real-time assessment of migratory behaviors and morphology of the cells under investigation. WO2016129894A1 discloses an apparatus with a unidirectional texture on a first side of an ion-permeable surface layer to improve fluid flow in the case of electro-osmosis.

### SUMMARY

The present invention relates generally to the field of cell migration assay platforms (CMAPs), including: devices and methods for characterizing migratory behaviors and morphology of cells *in vitro* through measurement and analysis of changes in electrical impedance that correlates to changes in spatial coverage and migratory behaviors of cells as the cells are allowed to grow and proliferate on or over highly directional textured surfaces that mimics the extracellular microenvironment *in vivo;* devices and methods for guiding behaviors and morphology of cells *in vitro* using cell confinement structures and highly directional textured surfaces; and devices and methods for guiding and characterizing behaviors and morphology of cells *in vitro* through measurements of changes in electrical impedance, also utilizing cell confinement structures and highly directional textured surfaces.

In an aspect, the present invention provides topographically-guided cell migration devices having a substrate, an ion-permeable surface layer having a unidirectional textured first side, and a pair of electrodes having interdigitated electrode digits disposed between the substrate and the ion-permeable surface layer. The unidirectional texture of the ion-permeable surface layer is configured to guide migration of a cell culture in a substantially linear migration direction. The cell migration devices may form part of a cell analysis system that includes a workpiece configured to securely hold the device and to transmit an electrical current between pair of electrodes.

In another aspect, the present invention provides topographically-guided cell migration devices having a substrate, a surface layer having a unidirectional textured first side, and a cell confinement structure with two walls that run substantially parallel to the unidirectional texture in order to guide migration of the cell culture in a substantially linear migration direction. The cell migration devices may form part of a cell analysis system that includes a workpiece configured to securely hold the device, for imaging of the cell culture deposited upon the textured first side of the surface layer.

In another aspect, the present invention provides methods for analyzing migration of a cell culture. The methods include seeding a first area of an ion-permeable surface layer with the cell culture, the ion-permeable surface layer having a unidirectional texture formed on a first surface thereof; guiding, with the unidirectional texture, a migration of the cell culture from the first area to a second area of the ion-permeable surface layer in a substantially linear migration direction; transmitting an electrical current between a first electrode and a second electrode that are positioned beneath the unidirectional texture and proximate to the ion-permeable surface layer, wherein a plurality of digits of the first electrode is interdigitated with a plurality of digits of the second electrode; measuring an impedance to the electrical current; and determining, based on the measured impedance, a migration distance of the cell culture from the first area toward the second area.

This summary is provided to introduce a selection of concepts in a simplified form that are further described below in the Detailed Description. This summary is not intended to identify key features of the claimed subject matter, nor is it intended to be used as an aid in determining the scope of the claimed subject matter.

### DESCRIPTION OF THE DRAWINGS

The foregoing aspects and many of the attendant advantages of the present invention will become more readily appreciated as the same become better understood by reference to the following detailed description, when taken in conjunction with the accompanying drawings, wherein:
FIGURE 1 illustrates operating principles of cell migration assay platforms, according to an aspect of the present disclosure.
FIGURE 2A shows an exemplary layout of interdigitated electrode structures formed on a substrate, according to an aspect of the present disclosure.
FIGURE 2B shows a closeup schematic view of an exemplary interdigitated electrode of FIGURE 2A.
FIGURE 3 shows exemplary interdigitated electrode structures in varying electrode widths and spacings, each of which interdigitated electrode structures can be used in implementing cell migration assay platforms of the present disclosure.
FIGURE 4 shows a schematic closeup view of the interdigitated electrode structures of FIGURE 3.
FIGURE 5A illustrates an exemplary process for fabricating a textured first side of a surface layer over interdigitated electrodes, according to another aspect of the present disclosure.
FIGURE 5B shows atomic force microscopy (AFM) representations of three-dimensional topography of a nanotextured surface layer and its dimensional representations in a graphic form, shown in AFM fluid mode while immersed in phosphate-buffered saline.
FIGURE 6 shows an exemplary method for implementing a cell migration assay platform according to an aspect of the present disclosure.
FIGURE 7A shows an exemplary cell confinement structure and method according to another aspect of the present disclosure.
FIGURE 7B shows another exemplary cell confinement structure and method according to another aspect of the present disclosure.
FIGURE 7C shows another exemplary cell confinement structure and method according to another aspect of the present disclosure.
FIGURE 7D shows another exemplary cell confinement structure and method according to another aspect of the present disclosure.
FIGURE 7E(i) shows a cell-seeding region of the present disclosure. FIGURE 7E(ii) shows a top view of an exemplary roof structure mated with an cell migration assay platform of the present disclosure. FIGURE 7E(iii) and FIGURE 7E(iv) show closeup top views of selected wells.
FIGURE 7F shows another exemplary cell confinement structure and method according to another aspect of the present disclosure.
FIGURE 8 shows another exemplary method for confining a cell-seeding region prior to initiating cell migration according to another aspect of the present disclosure.
FIGURES 9A, 9B and 9C show a cross-sectional view of various insert structures according to another aspect of the present disclosure.
FIGURE 10A shows an exemplary cell confinement structure according to another aspect of the present disclosure.
FIGURE 10B illustrates an acellular region produced by a cell confinement structure according to another aspect of the present disclosure.
FIGURE 11 illustrates a cell analysis system according to another aspect of the present disclosure.
FIGURE 12A illustrates an upper perspective view of a workpiece according to another aspect of the present disclosure. FIGURE 12B illustrates a lower perspective view of the workpiece of FIGURE 12A. FIGURE 12C illustrates an exploded view of the workpiece of FIGURE 12A. FIGURE 12D illustrates a side view of the workpiece of FIGURE 12A.
FIGURE 13A illustrates different parallel and perpendicular configurations of the interdigitated electrodes in reference to the longitudinal direction of patterns formed on the surface layer, according to another aspect of the present disclosure.
FIGURE 13B displays normalized impedance measurements taken at 10 kHz using the nanotextured plates of the present disclosure implemented in parallel and perpendicular electrode configurations, as compared to the same measurements obtained on a flat (or untextured) plate.
FIGURE 14A illustrates impedimetric cell migration studies showing how nanotopography of cell surface layers of the present disclosure relates to cellular migration speed and the resistance to mitomycin, a known cellular proliferation inhibitor.
FIGURE 14B shows how cell migration distance across cell migration assay platforms of the present disclosure correlates with impedance values measured with an exemplary apparatus of the present disclosure.
FIGURES 15A and 15B illustrate how PIK3CA knock-in MCF10A cells exhibit responses in impedance value to different dosages of the PI3K inhibitor LY294002 on untextured (FIGURE 15A) and nanotextured (FIGURE 15B) surfaces of the present disclosure.
FIGURE 15C illustrates a comparative endpoint dose-response study showing enhanced resistance of the cells to the compound LY294002 when the cells are directed to migrate on a nanotextured surface of the present disclosure vs. a flat surface.

One skilled in the relevant art will appreciate that elements in the figures may be illustrated for simplicity and clarity and have not necessarily been drawn to scale. For example, the dimensions of some of the elements in the figures may be exaggerated relative to other elements to make various embodiments of the present disclosure easily understood. Also, common but well-understood elements that are useful or necessary in a commercially feasible embodiment are often not depicted in order to highlight the key features of these various embodiments of the present disclosure.

### DETAILED DESCRIPTION

Examples of devices, systems, and methods are described herein to describe various embodiments of cell migration assay platforms (CMAPs). In the following description, numerous specific details are set forth to provide a thorough understanding of the examples. One skilled in the relevant art will recognize, however, that the techniques described herein can be practiced without one or more of the specific details, or with other methods, components, materials, etc. In other instances, well-known structures, materials, or operations are not shown or described in detail to avoid obscuring certain aspects.

References throughout this specification to "one example" or "one embodiment" or the like means that a particular feature, structure, or characteristic described in connection with the example is included in at least one example of the present disclosure. Thus, the appearances of the phrases "in one example" or "in one embodiment" in various places throughout this specification are not necessarily all referring to the same example. Furthermore, the particular features, structures, or characteristics may be combined in any suitable manner in one or more examples.

In view of many features and advantages offered by the present invention it will be evident to those skilled in the relevant art that various embodiments of the present invention and their modifications could be used in various cell migration, adhesion, proliferation or morphology studies. For example, devices of the present invention may be implemented as CMAPs by including a highly texturized surface layer and one or more optional interdigitated electrode pairs. As another example, devices of the present invention may be implemented as CMAPs by including a highly texturized surface layer and an optional cell confinement structure (such as a well or microfluidic channel) that confines cells within a specific cell-seeding region, separated from an acellular region into which the cells can migrate (e.g., upon removal of a migration barrier). Such embodiment may or may not include one or more optional interdigitated electrode pairs. The cell confinement structures also serve to guide migration of the cells in a substantially linear direction from the cell-seeding region toward the acellular region.

In an aspect, CMAPs of the present invention are configured to characterize migratory behaviors and morphology of cells under investigation by measuring the real-time changes in electrical impedance between an electrode pair, the changes correlating to the changes in spatial coverage and migratory behaviors of cells on or over the electrodes as the cells are allowed to expand and migrate unidirectionally in an *in vitro* setting that mimics the extracellular microenvironment *in vivo.* In another aspect, CMAPs of the present invention are configured to confine migratory behavior and proliferation of cells under investigation to a substantially linear migration direction utilizing cell confinement structure, for example wells and microfluidic channels. Accordingly, CMAPs of the present invention may be configured to measure changes in electrical impedance of cells, and/or to confine migratory behavior utilizing cell confinement structures. Any CMAP of the present disclosure having wells may be implemented in a single-well format or a multi-well format, e.g., 24-well, 48-well, 96-well, or other multi-well format.

The present invention overcomes various limitations of the conventional 2D and 3D assay models by providing a cell interface surface with textured topography (e.g., nanotextured topography) that creates quasi-3D environments for cell adhesion, proliferation and migration. For example, the cell interface surfaces patterned with unidirectional nanogrooves recapitulate the nanotopography of the extracellular matrix that helps guide cancer cells from their tumors of origin; thus, the cells migrating *in vitro* on such nanotextured surfaces display higher migration speeds and reduced directional variances. Also, their behaviors and morphologies match more closely those found in 3D matrices and tissue explants. Further, *in vitro* migration phenotypes of patient-derived cancer cells measured or detected with such nanotextured cell interface surfaces can serve as surrogates to predict tumor recurrence in cancer patients. These findings present a compelling scientific basis for applying the devices, systems and methods of the present disclosure to screen various cancer therapeutics.

Previously, microscopy-based cell tracking was a key component of most conventional CMAPs. However, the microscopic cell tracking and the quantification of data therefrom in a multi-well experimental setting require numerous hours of hardware operation and complex data analysis. Such approach also suffers from relatively low temporal resolution and often necessitates chemical labeling of the cells. Microscopes are also prone to mechanical failures and spontaneous defocusing in long-term, automated experiments. Electrical impedance-based assay platforms, in which cells are subjected to high-frequency, oscillating electrical currents and the degrees to which cells impede such current flows thereof are analyzed in real time, do not suffer from such shortcomings.

However, a few existing impedance-based cell-migration assay devices suffer from limitations that are inherent in their designs. U.S. Patent 7,470,533 by Xu describes a device, wherein cells initially confined by a removable barrier are, upon removing the barrier, directed to spread radially and migrate over a surrounding, concentric detection region. As cells migrate outward radially on or over the interdigitated electrodes in the detection region, the changes in impedance between or among the electrodes are analyzed to detect the changes in cell morphologies. U.S. Patent 8,227,223 by Giaever describes the inverse of this approach, wherein cells are initially excluded from a circular active electrode as a pulsed electric field generated therefrom prevents cell adhesion thereon. Upon discontinuing the electrical pulse, cells are directed to migrate radially inward on or over the circular electrode and the resulting changes in electrical impedance are analyzed to monitor migration of the cells. U.S. Patent Application 12/288,971 by Wang discloses a similar inward radial migration assay device, wherein nonfouling self-assembled monolayers are applied on the surface of the electrodes to inhibit cell adhesion. These monolayers desorb when an electrical stimulus is applied thereunto, thereby allowing the cells in the outlying region to migrate inwardly and the corresponding changes in impedance be measured. Importantly, none of these prior art devices are configured to induce highly directional migration of cells in a pro-migratory biomimetic microenvironment. Such directionally induced migratory anisotropy cannot be effectively recapitulated by the prior art radial cell-migration assay devices.

In an aspect, the present invention improves the prior art by providing a unidirectionally patterned cell-interface surface layer embedded with directionally oriented interdigitated electrodes in such a way that, as cells are allowed to grow and proliferate directionally within an environment more reminiscent of that in which they exist *in vivo,* the migratory behaviors of the cells can be accurately determined by measuring the real-time changes in electrical impedance. In particular, the electrodes are directionally configured to effectively detect the behaviors of the directionally migrating cells. In addition, the present disclosure improves the CMAP's predictiveness and efficiency by recapitulating the biophysical microenvironment which the cells are subjected to.

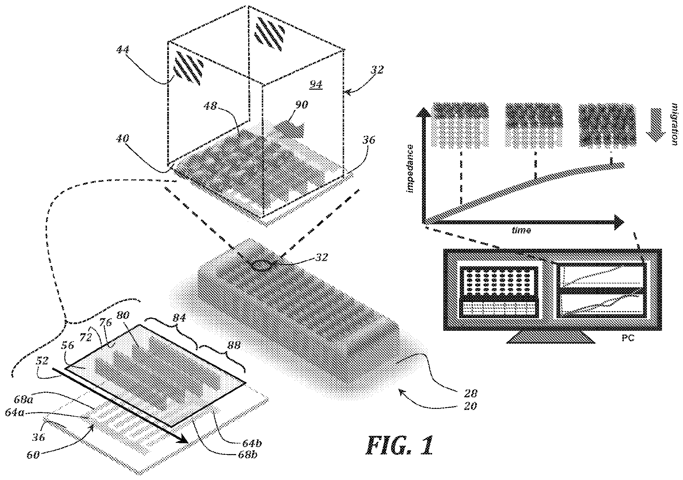

FIGURE 1 illustrates some basic configurations and operating principles of an aspect of the present disclosure, i.e., a cell migration assay platform ("CMAP") 20 that is configured to measure changes in electrical impedance of cells. According to the nonlimiting embodiment shown in FIGURE 1, the CMAP 20 is implemented in a multi-well housing 28 containing an array of cell confinement structures 32 (in this embodiment, wells 32) disposed on a substrate 36. The cell confinement structures 32 are optional. In the illustrated embodiment, each well 32 has a bottom area 40 bounded by at least two walls 44 that guide migration of a cell culture 48 in a longitudinal direction 52 of a textured surface layer 56 (described below). The substrate 36 may include an adhesive layer that bonds the housing 28 to the substrate 36 and fluidically seals each well 32 from adjacent wells 32. The housing 28 and the underlying adhesive layer are configured to be positioned over, and specifically aligned with, particular points or areas on the substrate 36. The substrate 36 may be made in a relatively thick insulating layer of polycarbonate, polyethylene terephthalate, glass or similar biocompatible materials.

The substrate 36 has a plurality of electrode structures 60 deposited thereon, each electrode structure 60 comprising at least a pair of interdigitated electrodes (IDEs) 64a and 64b, with each interdigitated electrode having a plurality of electrode digits 68a and 68b, respectively (referred to as digits herein). The digits 68a of the first electrode 64a are interdigitated with the digits 68b of the second electrode 64b. As used in this application, "interdigitated" may mean interlocked, i.e., overlapping in two dimensions (e.g., a x-dimension and a y-dimension). The digits 68a and 68b of the first and second electrodes 64a and 64b, respectively, are disposed in the bottom area 40 of the well 32. Such placement of electrode digits relative to a bottom area of a well may be replicated in any embodiment of the present disclosure.

In an embodiment, a layer of insulating material such as SU8 may be patterned over portions of the electrode structures 60. The electrode structures 60 may constitute any electroconductive material, such as gold, titanium oxide, or indium tin oxide, and may include an underlying layer that facilitates deposition of the electrode structures 60 on the substrate 36. The interdigitated electrodes 64 are configured such that oscillating electrical current can be transmitted between and among the digits 68, such that any interference to the current caused by any material(s) interposed between the digits 68 can be quantified as the electrical impedance of the material.

The surface layer 56 is formed on and over at least a portion of the substrate 36 and the electrode structures 60 thereon. The surface layer 56 has a highly directional textured first side 72 that faces away from the substrate 36, and a second side 76 that is disposed proximate to the substrate 36. In FIGURE 1, the textured first side 72 is patterned in a form of highly directional grooves 80 which are elongated, nano- and/or micro-scale structures running substantially parallel to each other in a periodically repeating manner. The periodicity of such nano- or micro-grooves ranges between about 0.1 µm and about 2 µm in cross sectional horizontal length. The textured first side 72 need not conform precisely to the area of the substrate 36 where the electrode structures 60 are deposited. In addition to substantially parallel grooves, other unidirectional patterns are contemplated. The directionality of the texture has a high level of prominence such that it may be considered unidirectional. As used herein, the term "unidirectional" in reference to textured surface layer means a surface having a texture that exhibits a single predominant texture direction and/or a substantially parallel alignment of features, even if the texture may also exhibit another direction(s) in a substantially lesser degree. Unidirectional may also mean "substantially linear."

In some embodiments, the widths and heights of, and the spacing between, the grooves need not be uniform over the entire textured side of the surface layer. Rather, one or both dimensions may be configured to vary in whole or part across the textured side in a way that optimizes cellular responses, and migratory behaviors resulting therefrom, to a desired topographic configuration that is suited for different cell types or different assay applications. For example, in an embodiment, periodically repeating nanoscale grooves may be divided into a plurality of separately functioning groups of nanogrooves, with each groove of a first group having a different height and/or a different width relative to each groove of a second group. In another embodiment, each groove of the first and second groups may have common heights and/or widths. In an embodiment, the first and second groups may be separated by a dividing path formed in the surface layer (e.g., a micro- or nano-scale groove or wall formed in the surface layer having a larger width and/or height). In an embodiment, the dividing path is unavailable for cell attachment.

In operation, the cells 48 are seeded on a cell-seeding region 84 of the textured first side 72 of the surface layer 56, the cell-seeding region 84 being located proximal to an acellular region 88 of the textured first side 72 of the surface layer 56. The cell-seeding region 84 and the acellular region 88 are located in the bottom area 40 of the well 32 and exposed to a cavity 94 of the well 32. Such placement of the cell-seeding region 84 and acellular region 88 relative to the bottom area 40 of the well 32 may be replicated in any embodiment of the present disclosure.

In FIGURE 1, the cell-seeding region 84 and the acellular region 88 are distinct locations on the same textured first side 72 of the surface layer 56, but may not otherwise have different properties. In an embodiment (described below), the cell-seeding region 84 may be physically distinguished from the acellular region 88 by a removable insert or other structure. The seeded cells 48 sense the alignment of the highly textured first side 72 and, as the cells 48 migrate, grow and proliferate, the cells 48 expand along the longitudinal axis 52 of the unidirectional texture with increased velocity in response to the topographical cues. The longitudinal axis 52 of the unidirectional texture generally corresponds with the prominent direction of the texture. Further, the longitudinal axis 52 of the unidirectional texture generally guides cell migration along the longitudinal axis 52, and therefore corresponds with a migration direction 90 of the cells. Because of the linearity of the unidirectional texture, the migration direction 90 of individual cells within the migrating population is itself substantially linear. As used herein, the term "substantially linear" in reference to cell migration direction means a predominantly linear direction, e.g., when cells migrate in one direction that significantly predominates, even though the cells may also migrate in another direction(s) in a substantially lesser degree.

The embodiment of FIGURE 1 may be utilized by applying an electrical voltage or current at a selected frequency between the interdigitated electrodes 64a and 64b to produce oscillating current flows between the adjacent digits 68a and 68b. As the cells 48 migrate directionally across the textured first side 72 of the surface layer 56 and across the spatially distributed interdigitated electrodes 64a and 64b, the current flowing between the adjacent electrode digits 68a and 68b is further impeded with increasing cell coverage on or over the interdigitated electrodes 64a and 64b. The resulting changes in amplitude and time delay in the current flows, in the case of an applied voltage or voltage between the electrodes in the case of an applied current, cause changes in the measured impedance that can be correlated to the distance or area covered by the collectively migrating cells 48 under investigation.

Impedance is represented as a complex value, and as such the real and imaginary components, in addition to the magnitude and/or phase angles of the impedances and the changes thereof may be read by an impedance analyzer having a processor and a data store programmed with one or more logic modules (e.g., computer programs) that are configured to quantify cell positions (e.g., in real time), speeds of cell movements, and other migratory behaviors. Such impedance analyzers are described below. The experimental data obtained from CMAPs of the present disclosure confirm that greater migration distances produce correspondingly greater electrode impedances in a highly linear relationship.

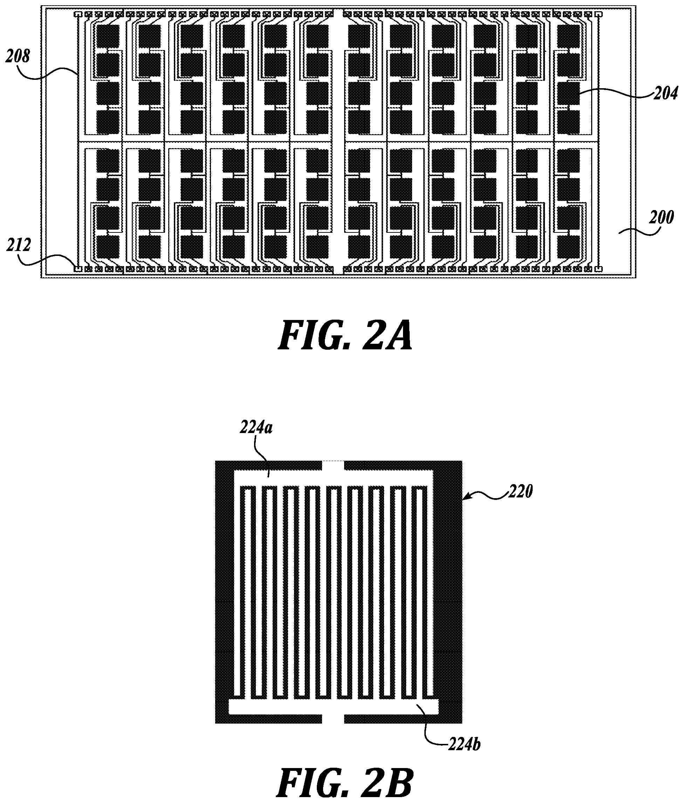

FIGURE 2A illustrates an electrode layout on a substrate 200 configured for use as part of a 96-well CMAP comprising an array of 96 electrode structures 204 (shown in black rectangles) that are constructed as described with respect to FIGURE 1. Each electrode structure 204 comprises at least one pair of interdigitated electrodes (IDEs) having a plurality of digits that are connected to electrical traces (such as trace 208) routed to the opposing sides of the substrate 200, where they terminate in contact pads (such as contact pad 212), which can be configured to interface with external circuitry such as an impedance analyzer. The illustrated embodiment is merely exemplary. In an embodiment, each electrode structure may include more than one pair of interdigitated electrodes. For example, an embodiment may include electrode structures, each having two or more pairs of interdigitated electrodes for use in a single well of a CMAP. Other embodiments may include more than one type of electrode. For example, an embodiment may include both interdigitated electrodes as described herein and other electrode types, e.g., in the same well. In such embodiments, the different electrodes may operate independently of each other; for example, interdigitated electrodes may be utilized to measure cell migration, while a second electrode type may be used for cell stimulation or another purpose. Embodiments of the present invention that include one or more cell confinement structures as described below may have one or more electrode structures that do not have interdigitated electrode pairs, such as the electrodes shown in International Application No. PCT/US18/26534, which is herein incorporated by reference in its entirety.

FIGURE 2B illustrates a schematic closeup view of an electrode structure 220, which is similar to electrode structure 204 of FIGURE 2A. Electrode structure 220 comprises two opposing, interdigitated electrodes 224a and 224b in a configuration suitable for use on a substrate of the 96-well CMAP of FIGURE 1. In the schematic of FIGURE 2B, the white area corresponds to the electrodes 64 and electrode digits 68 of FIGURE 1. This electrode structure 220 is designed to eliminate the need for a designated counter electrode, thereby maximizing the area for cell migration, and to enhance linearity in the relationship between the measured changes in impedance and the migration distances of the cells.

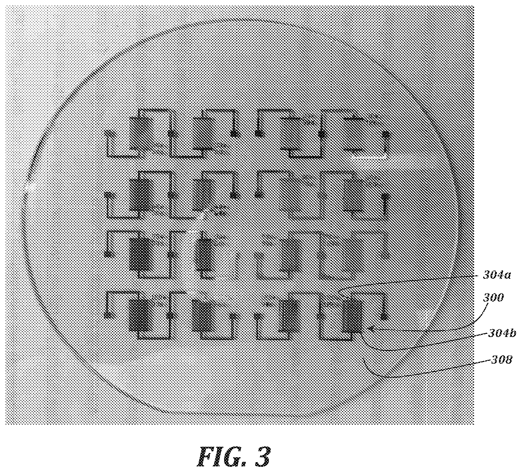

FIGURE 3 shows exemplary electrode structures 300, each of which are similar to the electrode structures 204 and 220 of FIGURES 2A and 2B, respectively. Each electrode structure 300 having a pair of interdigitated electrodes 304a and 304b. The interdigitated electrodes 304 have digits with varying widths (30, 60, 90 and 120 µm) and spacings (30, 60, 90 and 120 µm), and are fabricated in Cr/Au on a glass substrate 308. Each electrode structure 300 has a construction similar to the electrode structures 60 and 204 of FIGURES 1 and 2B, respectively. Electrode structures 300 exhibiting any of these specific widths and spacings may be fabricated using photolithography, inkjet/screen printing, laser ablation, and/or other similar processes, and can be used in implementing the CMAPs of the present disclosure. The electrode widths and spacings shown in FIGURE 3 are exemplary. In other embodiments, the electrode widths and spacings can differ from the values of FIGURE 3, for example, to produce optimal sensitivity in measuring impedimetric effects.

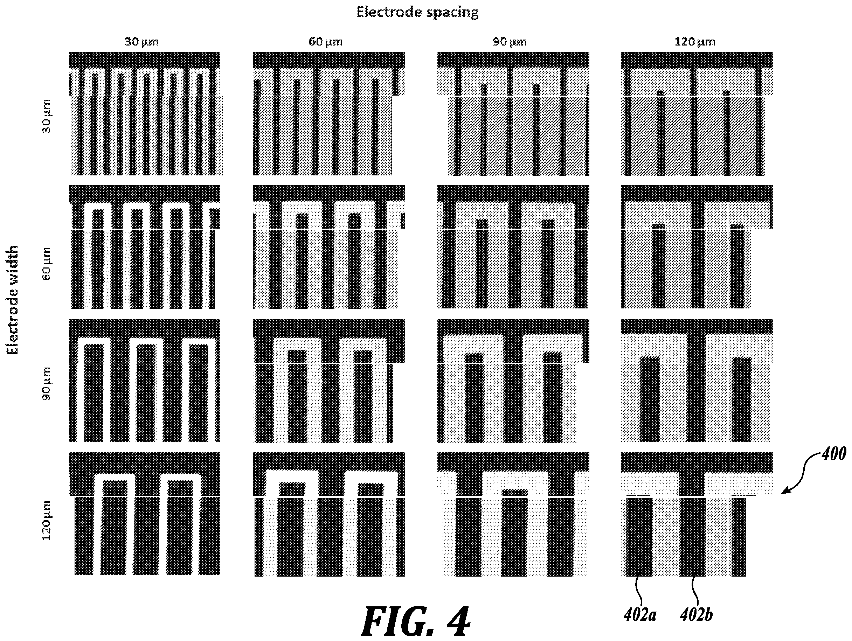

FIGURE 4 shows a closeup schematic view of electrode structures 400, which are similar to the electrode structure 300 of FIGURE 3. The areas marked in black represent the areas where Cr/Au or other material is patterned. Each electrode structure 400 comprises a pair of interdigitated electrodes, each of which comprise a plurality of digits 402 (e.g., digits 402a and 402b), respectively. The digits 402 have the same spacings and digit widths as the digits of electrodes 304 of FIGURE 3. The first row of electrode structures 400 have 30 µm-width electrode digits with 30, 60, 90, 120 µm spacings. The second row of electrodes structures 400 have 60 µm-width electrode digits with 30, 60, 90, 120 µm spacings. The third row of electrode structures 400 have 90 µm-width electrode digits with 30, 60, 90, 120 µm spacings. The fourth row of electrode structures 400 have 120 µm-width electrode digits with 30, 60, 90, 120 µm) spacings. In an embodiment, the digits 402 each have a 120 µm width, with adjacent digits 402 having 120 µm spacing; this combination provides lower noise values and higher dynamic range values as compared with other combinations. In other embodiments, electrode spacing and electrode width may each be as wide as up to about 300 µm. e.g., 150 µm, and as narrow as about 10 µm.

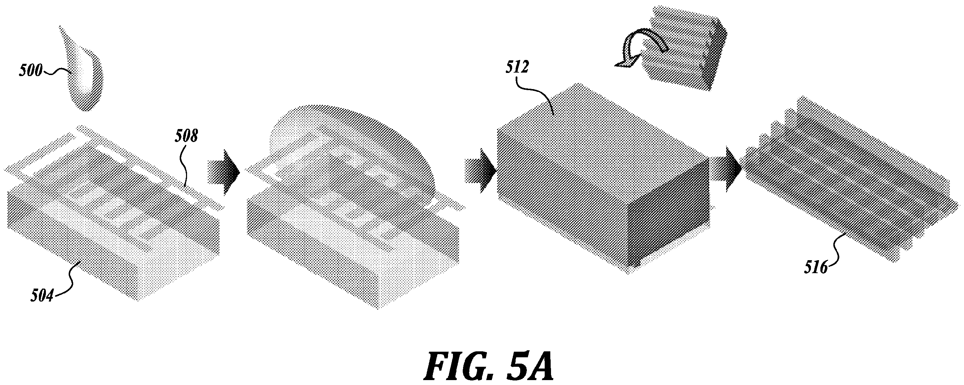

FIGURE 5A illustrates an exemplary process for fabricating a highly directional textured first side of a surface layer over interdigitated electrodes to produce a CMAP. While the embodiment of FIGURE 5A includes electrodes, such a process may also be utilized to fabricate a highly directional textured first side of a surface layer in embodiments without interdigitated electrodes, e.g., embodiments designed to facilitate monitoring of cell migration through other means, e.g., visual observation. In the embodiment of FIGURE 5A, a drop of a polymer resin 500 (such as Nafion) is applied on a substrate 504 having interdigitated electrodes 508 preformed thereon. Masks (not shown) may be applied to certain portions of the substrate 504 in a desired configuration. A stamp or mold 512 with a footprint having the negative of a desired unidirectional texture is pressed onto the droplet of the polymer resin 500 deposited atop the electrodes 508 such that the polymer resin 500 covers the entirety of the electrodes 508. The composition of the polymer resin 500 is optimized such that the capillary force between the stamp 512 and the resin 500 draws the resin 500 into the topographic features of the stamp 512, where it cures in time. Once the polymer resin 500 is cured, the stamp 512 can be removed, leaving a polymer surface layer 516 with a 3D morphology that approximates the negative of the stamp's topography. Any masks can then be removed, leaving the isolated textured first side of the surface layer 516.

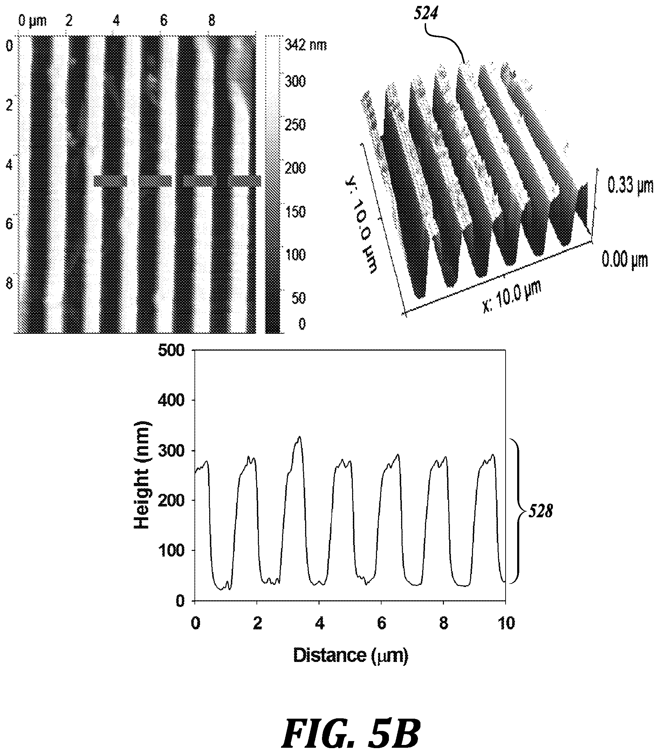

In CMAP embodiments having interdigitated electrodes, the material used to form the textured first side of the surface layer (i.e., the resin 500 in FIGURE 5) should be water-permeable and ion-permeable in order to minimize the interference of the material with electrical excitation signals applied through the underlying electrodes. The ion-permeable nature of the surface layer enables efficient detection of electrical current flows by the underlying electrodes not in physical contact with the overlying cells or analytes. Thus, the currents detected by the electrodes underlying the ion permeable surface layer can be relayed to an external circuitry such as an impedance analyzer for further analysis. Given its excellent ionic properties and superior thermal and mechanical stability, Nafion, including commercially available Nafion stock solution compositions comprising 5% or 20% Nafion, is a suitable material for the polymer surface layer as it enables reliable electrical measurements from the underlying electrodes while retaining the rigidity required to form high-fidelity nanoscale 3D topographic structures. Nafion is particularly suitable because its conductance increases when Nafion-based surface layers are imparted with a texture, e.g., the nanopatterns described herein. Nafion is further suitable because its conductance increases in highly humid or wet environments such as cell culture applications. Thus, utilizing a highly texturized Nafion surface layer in CMAPs having interdigitated electrodes may lead to better data produced by those CMAPs. However, other ion permeable materials such as gelatin, methacrylated gelatin, peg diacrylate gels, thermoplastic track-etched membranes, Polyethylene Terephthalate Glycol (PETG), MATRIGEL^{®}, Poly-Acrylamide, Poly N-isopropylacrylamide (Poly-NIPAM), agarose gels, dextran gels, and any other crosslinked hydrogel or polymer electrolyte may be used, instead of Nafion or in combination with Nafion or one or more of the above-described materials, with similar processes to utilize different advantages afforded by selected material(s) that are more suitable for different applications.

In an embodiment, a Nafion composition is patterned directly onto the substrate of the plate by capillary force lithography, using a nanotextured polydimethylsiloxane (PDMS) mold. Nafion patterning may be applied either before or after affixing a multiwell housing onto the substrate. Nafion is typically dispersed in amphiphilic solvents that can infiltrate even nanoscale topographies in molds made from a variety of materials. Nafion compositions can be cast very thinly using this method, to thicknesses of a few µm. It is generally desirable to minimize the thickness of the polymer surface layer so that electrical current flows through analytes between the electrodes are not significantly affected by the presence of the polymer material. In an embodiment, a Nafion composition surface layer has thicknesses between about 1 µm and about 5 µm. Nafion composition cures by solvent evaporation, and thus can be processed independently of additional stimuli or chemicals. Furthermore, Nafion is naturally cell-and protein-adhesive and thus requires no treatment to render it as such, making it an ideal tissue culture substrate for cell-based assay platforms.

FIGURE 5B shows atomic force microscopy (AFM) representations of 3D topography of a Nafion-patterned surface layer 520 and its dimensional representations in a graphic form, shown in AFM fluid mode while immersed in phosphate-buffered saline. It is seen that Nafion nanostructures 524 fabricated using the processes described in FIGURE 5A closely recapitulate anisotropic geometric features and dimensions of extracellular matrix fibers found in tumor-invasive microenvironments *in vivo.* In particular, the Nafion nanostructures 524 form a plurality of substantially parallel grooves 528. In the illustrated embodiment, each groove has a height (depth) of between about 200 nm and about 400 nm. Other embodiments may include one or more grooves having a height ranging from about 100 nm to about 1,000 nm.

CMAPs having interdigitated electrodes formed on a substrate may utilize the highly directional texturized surface layer to facilitate measurement of cell migration. For example, a CMAP may utilize substantially parallel grooves (such as the grooves 528 of FIGURE 5B), which have a longitudinal direction. The grooves (and the longitudinal direction thereof) may be fabricated to run either parallel or perpendicular to the longitudinal axes of the digits of the interdigitated electrodes (as shown in FIGURE 1, for example). This way, a migration distance of cells across the surface layer (in a migration direction) varies substantially linearly with the cell's impedance to the current transmitted between the interdigitated electrodes. Thus, the relative orientation of the surface layer can affect how the excitation current interferes with the overlying cells or tissue and thus can offer different advantages for different applications.

Generally, the housing of the CMAP may be monolithic and processed as a whole, or subdivided into separate sub-housings, which can be processed individually and then combined, or combined and processed as a whole. In an embodiment, the housing may include a plurality of cell confinement structures (e.g., wells) that are positioned to align with rectangular-shaped electrode structures of the interdigitated configuration. In an embodiment, the wells are substantially formed from a bottomless thermoplastic housing that is adhered to the substrate using a double-sided adhesive film having cutouts for openings that expose the textured first side of the surface layer. The wells and the corresponding openings of the adhesive substrate may contain two or more right angles in the interior shape. This rectangular shape of the interior well structure is configured to direct cells to start migration at an equidistance one-dimensionally. The rectangular shape of the interior well structure also helps guide the migration of the cells along the longitudinal axis of the textured first side of the surface layer. In embodiments having interdigitated electrodes, the rectangular shape is configured to direct cells along either the longitudinal or transverse direction of the interdigitated electrodes, and contributes to the linearity between migration-induced increases in electrode coverage and the corresponding impedance increases be maintained according to the present disclosure. On the other hand, in the prior art assay devices that direct cells to migrate and proliferate to fill a circular region, the spatial coverage of the migrating cells increases in two dimensions, resulting in nonlinearity with respect to impedance changes and thereby making it difficult to determine migration distances or speeds of the cell culture from the measured impedance changes.

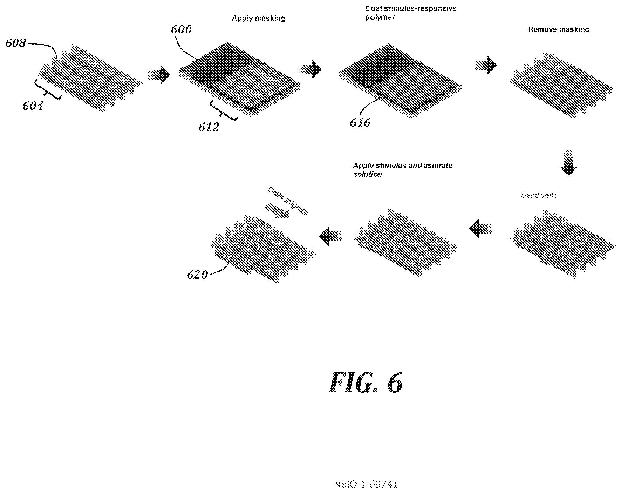

FIGURE 6 shows an exemplary CMAP structure and method for implementing a CMAP, wherein a stimulus-responsive polymer is coated over an area on the nanotextured surface layer as a means for creating an acellular region thereon. In FIGURE 6, a mask 600 is applied onto a desired cell-adherent region 604 on a nanogrooved surface layer 608. The mask 600 may be fabricated from silicone elastomer, e.g., with cut-out openings for a cell migration region 612 where a polymer is to be coated. Then, a thermo- or electro-responsive polymer 616 is coated to the cell migration region 612 on the nanotextured surface layer 608. The polymer coating 616 can be applied by depositing aqueous polymer solutions and then evaporating the solutions. Upon removing the mask 600, cells 620 are seeded, and then the polymer coating 616 is dissolved away with a stimulus or otherwise removed (such as by lowering the temperature, or by applying an electrical current). Once dissolved solutions are aspirated, the cells 620 can migrate into the cell migration region 612 created. In an embodiment, a thermoresponsive polymer, such as poly n-isopropyl acrylamide (pnipam), is used for the polymer coating 616. This polymer is completely non-toxic and is highly soluble in water at temperatures below 32 degrees Celsius. As such, cells 620 seeded in a well containing a thermoresponsive polymer-coated cell migration region 612 can be allowed to adhere at physiological temperatures and then briefly exposed to a slightly lower temperature to dissolve away the coated polymer, creating an acellular region thereon. In an embodiment, the solution-cast polymer may be ablated in aqueous solution with external stimulus. This method may be implemented in CMAPs of the present disclosure, whether or not such CMAPs have interdigitated electrodes.

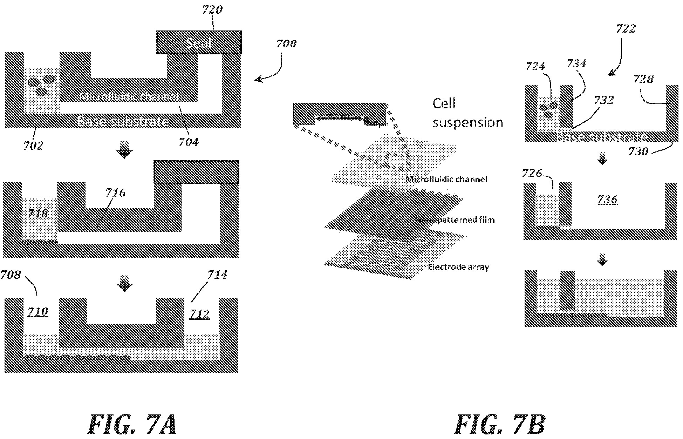

FIGURE 7A shows a schematic of a cell confinement structure 700 and method for implementing a CMAP having a highly textured surface layer (either with or without interdigitated electrodes), wherein the nanotextured surface layer and the electrodes deposited on a substrate 702 are enclosed within a microfluidic channel 704. The microfluidic channel 704 has a width ranging from 1,000 to 5,000 µm and a height ranging from 10 µm to 200 µm. An opening 708 for an inlet chamber 710 is provided at one end of the microfluidic channel 706. The inlet chamber 710 may coincide with a cell-seeding region of the highly textured surface layer. An outlet chamber 712 at the other end of the microfluidic channel 704 coincides with an acellular region of the surface layer and may also have an opening 714 but can be configured to be air permeable without a physical opening. The microfluidic channel walls (not shown) and ceiling 716 may comprise a same or different material. In use, a cell solution 718 may be seeded in the inlet chamber 710. The opening 714 at the outlet chamber 712 may initially sealed with a removable air tight seal 720 such that the surface tension of the cell solution 718 and the resulting air bubble occupying the acellular region form a barrier preventing cell migration to the acellular region. The opening 714 of the outlet chamber 712 can be unsealed at a desired time after introducing cell solution 718 into the inlet chamber 710. After the cell solution 718 is added and held in place by both the pressure of the atmosphere trapped in the sealed, solution-free outlet chamber 712 and the surface tension of the cell solution at the junction of the inlet chamber 710 and the microfluidic channel 704, the cells in the solution 718 attach to the highly textured surface layer over time. Migration can be initiated by unsealing the sealed opening 714 at the outlet chamber 712, thereby allowing influx of the cell solution 718 into the microfluidic channel 706. This influx may also be initiated by placing entire apparatus in a subatmospheric pressure environment, such as a vacuum chamber, or by adding a solution into the outlet chamber 712 to form a continuous fluidic connection throughout the microfluidic channel 706 between the two chambers, or any combination of the aforementioned techniques.

FIGURE 7B shows another cell confinement structure 722 and method for implementing a CMAP (either with or without interdigitated electrodes), wherein a cell solution 724 is added into a seeding chamber 726 of a well 728 having a highly textured surface layer formed on a substrate 730, wherein the cell suspension 724 is kept initially confined therein by a barrier formed by the surface tension formed at a microfluidic channel 732 underneath an insert 734 (which may be permanent) that separates the seeding chamber 726 (coinciding with a cell seeding region of the surface layer) from an outlet chamber 736 (coinciding with an acellular region of the surface layer). Unlike the outlet chamber in FIGURE 7A, the outlet chamber 736 in FIGURE 7B is open at the top. As the cell suspension 724 infiltrates through the microfluidic channel 732 to the outlet chamber 736 over time, cells attached to the cell seeding region of the surface layer commence migration towards the acellular region.

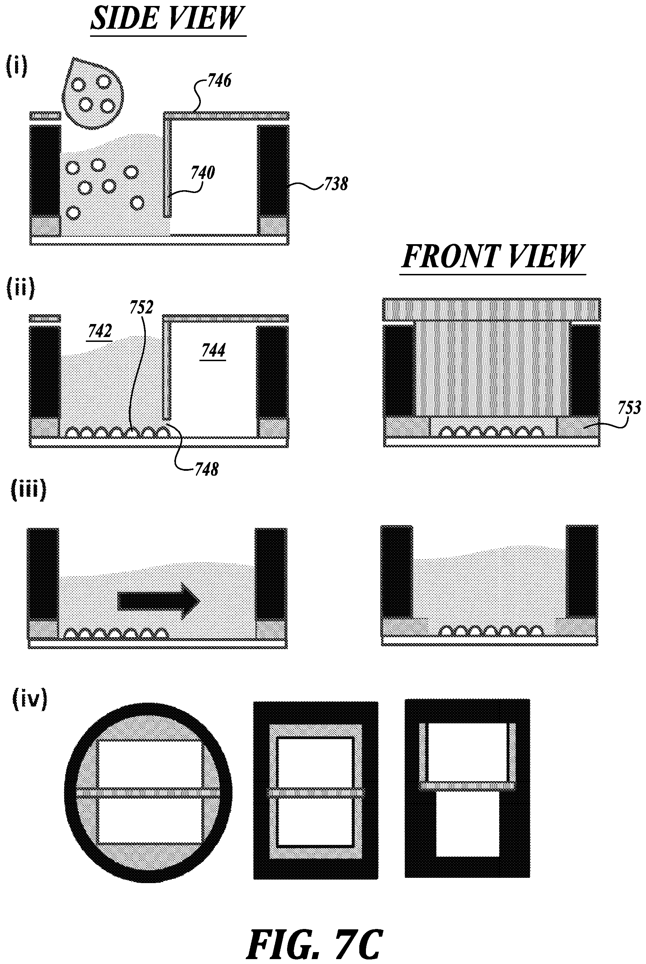

FIGURE 7C shows another exemplary cell confinement structure (in this example, a well 738) and method for implementing a CMAP (either with or without interdigitated electrodes), wherein an insert 740 that separates an inlet chamber 742 from an outlet chamber 744 is orthogonally integrated to a roof 746. The insert 740 projecting out of the integrated roof 746 can be inserted into the well 738 (i.e., a cavity of the well) to form a microfluidic channel 748 between the inlet chamber 742 and the outlet chamber 744. When the insert 740 is removed from the cavity 750, it allows cells 752 to migrate as they would in a conventional cell culture medium bath. FIGURE 7C(i) shows a cross sectional side view of the well 738 with this removable insert 740 in place. Cells 752 are seeded in the inlet chamber 742 (coinciding with a cell seeding region of the surface layer) and held in place by a barrier created by the surface tension it forms at the microfluidic channel 748. FIGURE 7C(ii) shows a cross sectional side and front views of the well 738 with cells 752 settling to the bottom of the well 738 (i.e., onto a highly textured surface layer) and forming a monolayer culture. FIGURE 7C(iii) shows the well 738 after the insert 740 is removed. The well 738 may comprise different geometries to make it suitable for a desired application. FIGURE 7C(iv) shows a cross sectional top view of embodiments of various well geometries and placements of the insert 740 of the present disclosure. In FIGURE 7C, the diagonally hatched area 753 represents a raised area having a microscale thickness that, in combination with the insert 740 affixed thereon, forms the walls of the microfluidic channel 748. These raised areas may either be part of the adhesive that bonds the bottomless multiwell housing to the substrate or may be physically integrated with the adjoining constructs of the well. These walls may be incorporated into any cell confinement structure of the present disclosure.

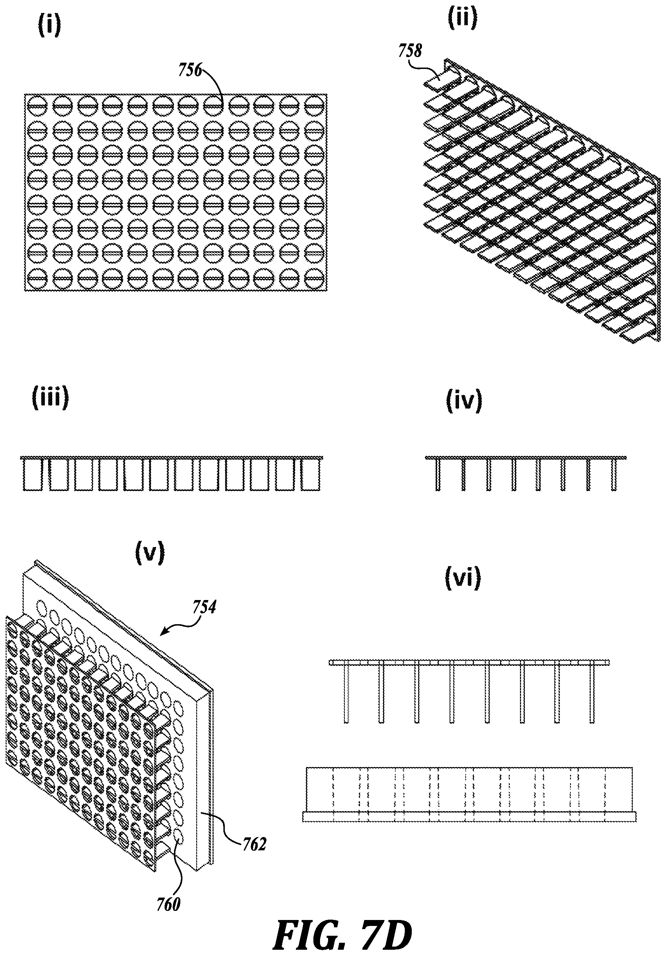

FIGURE 7D shows another exemplary cell confinement structure 754 and method for implementing a CMAP (either with or without interdigitated electrodes) comprising a roof 756 integrated with an array of 96 orthogonally projecting inserts 758, wherein each insert 758 is configured to be removably insertable into a corresponding well 760 of a 96-well housing 762, each well 760 having a highly textured surface layer located in a bottom area thereof. For the illustrated embodiment of FIGURE 7D and for other illustrated embodiments herein, the number of wells is merely exemplary and not intended to limit the scope of the present disclosure. FIGURES 7D(i), (ii), (iii) and (iv) show a top view, a perspective view, a front view and a side view, respectively, of the integrated roof 756 and insert 758 according to an embodiment of the present disclosure. FIGURES 7D(v) and (vi) show a perspective view and a side view of the integrated insert/roof structure 756, 758 positioned to be inserted into the 96-well housing 762.

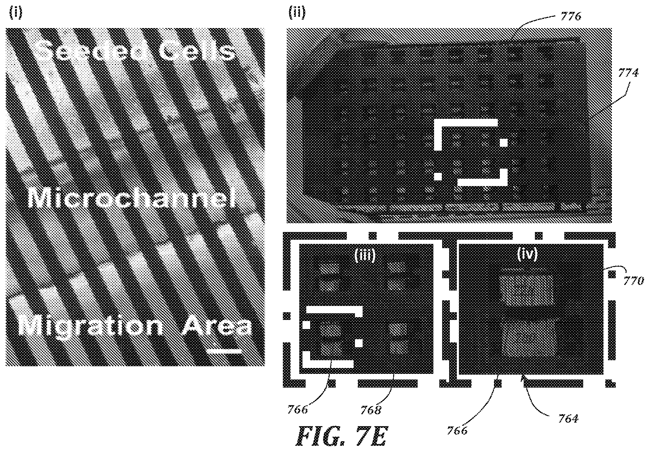

FIGURE 7E(i) shows another exemplary cell confinement structure 764 and method for implementing a CMAP (either with or without interdigitated electrodes) having a microchannel-forming insert 766 that is configured for removable insertion into a well 768 having a highly textured surface layer. Each well 768 includes an interdigitated electrode structure 770 located in a bottom area of the well, i.e., underneath a highly texturized surface layer. The interdigitated electrode structure 770 of FIGURE 7E has a construction consistent with FIGURES 1, 2B, 3, and 4. FIGURE 7E(ii) shows a top view of a 48-well roof structure 774 mated with a 48-well housing 776. FIGURE 7E(iii) and (iv) show closeup top views of selected wells 768 containing a cell-seeding chamber 778 and an acellular migration chamber 780 that are fluidically connected by a microfluidic channel formed by a gap between the insert 766 and the bottom area of the well 768.

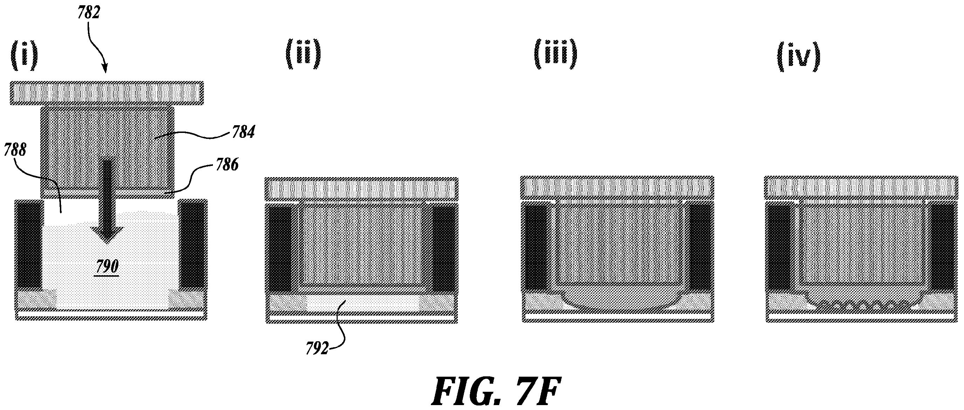

FIGURE 7F shows another exemplary cell confinement structure 782 and method for implementing a CMAP having a highly textured surface layer (either with or without interdigitated electrodes), wherein a removable insert 784 (such as shown in FIGURES 7C and 7D) is partially or completely over-molded with a porous overmold material 786 that swells upon prolonged contact with aqueous solutions. The overmold material 786 can be cast around and ultimately adherent to another material through chemical properties, friction, or mechanical interlocking. FIGURES 7F(i) and (ii) shows a cross sectional front view of an overmolded insert 784 being inserted into a cavity of a well 788 filled with a medium 790 such that it forms a microfluidic channel 792 underneath the insert 784. FIGURES 7F(iii) and (iv) show the overmold material 786 swelling in the presence of the medium 790, substantially obscuring the microfluidic channel 792 such that seeded cells cannot substantially pass through.

To avoid damaging the textured first side of the surface layer, the migration channels or inserts described in any of the embodiments in FIGURES 7A through 7F may be configured to not contact the textured first side of the surface layer at all or with any substantial force that could damage or disrupt the textured first side. Thus, a coating applied to the surface layer can remain effectively intact while the cell suspension is confined. Accordingly, the textured first side of the surface layer on which the monolayers migrate - may be coated with proteins prior to, during or after addition of cell suspension in order to influence cellular behavior.

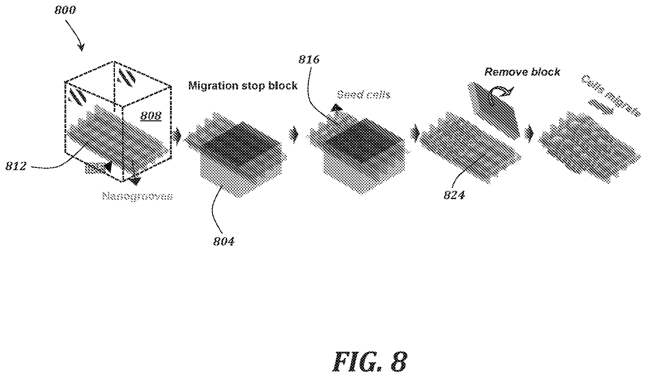

FIGURE 8 shows another cell confinement structure 800 and method for confining the cell-seeding region of the highly textured surface layer prior to initiating cell migration. An insert 804 (in this embodiment, an elastomeric stopping block 804) is placed on one side of a well 808 to form a watertight seal with a surface layer 812 such that a cell culture 816 seeded within a cell-seeding region that is unoccupied by the stopping block 804 remains confined therein, thus forming an acellular region 824 under the stopping block. The surface layer 812 is a polymer patterned in nanogrooves, as described above. Cell confinement can be reversed by removing the stopping block 804, such as with a removal apparatus. This removal apparatus may be attached to the stopping block 804 throughout the process or coupled to the block 804 only when its removal is desired. Pressure can be actively or passively applied to the stopping block 804 to ensure conformal contact between the stopping block 804 and the underlying surface layer 812 such that the cell culture 816 (especially if seeding a solution form) does not leak into the acellular region 824 underneath the stopping block 804. The illustrated embodiment is shown with an electrode structure as described above; however, this cell confinement structure 800 may be utilized with or without such electrode structures.

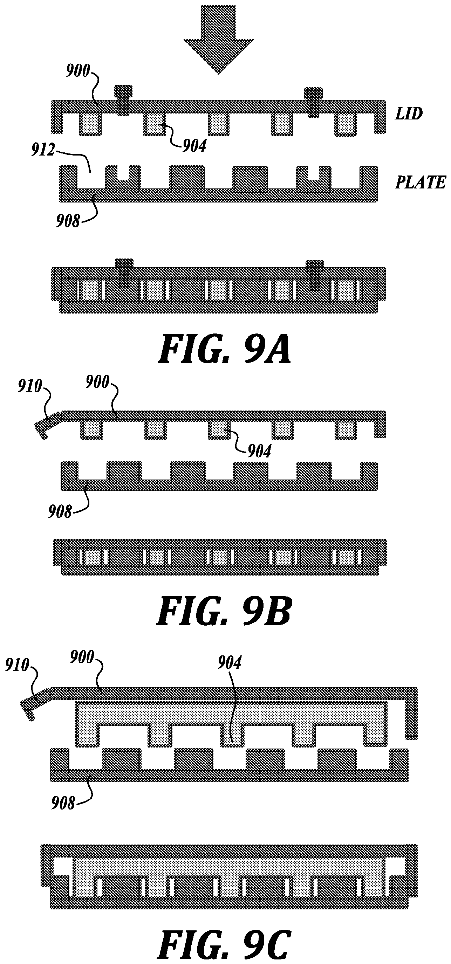

In an embodiment, an array of stopping blocks may be integrated into a lid or an insert structure composed one or more materials. The insert structure can be made compatible with a multiwell plate such that each of the stopping blocks can be inserted into each well in the multiwell plate. FIGURE 9A, 9B and 9C show a cross-sectional view of various insert structures comprising a lid and an array of elastomeric blocks that can be inserted within the corresponding array of wells in a multiwell plate. The blocks could be individually attached to a lid or integrated as a singular body of blocks that can be clamped or pressed down by the lid. In some embodiments, the presence of nanotopography on the acellular region may necessitate additional downward pressure be applied to the blocks in order to ensure an effectively fluid-tight seal with the acellular region. Therefore, the insert structure may be secured to the plate using screws, a living hinge, or other means of applying downward pressure to the blocks. FIGURE 9A shows a configuration wherein a lid 900 includes a plurality of inserts 904 (in this embodiment, stopping blocks 904), the lid 900 being bolted down to a plate 908 such the each insert 904 is inserted into a well 912. FIGURE 9B shows a configuration wherein the lid 900 is clamped to the plate 908 using a living hinge 910. The inserts 904 of any embodiment disclosed herein may incorporate ports through which a pipette or other means of depositing fluid can be inserted. In such embodiments, the lid 900 is configured to cover the inserts 904 to limit contamination and evaporation. FIGURE 9C shows another configuration using a living hinge, wherein the stopping blocks 904 are separate from the lid 900, yet interconnected to be placed independently as a unit for ease of handling. The stopping blocks 904 may be connected through another handling device, which may or may not incorporate overmolded rigid materials that can be used to improve accuracy and consistency in placing and aligning the stopping blocks 904 across multiple wells 912. In FIGURE 9C, the lid 900 can be clamped into the plate 908 to apply pressure to the underlying stopping blocks 904. The lid 900 in FIGURE 9C may also incorporate means (such as one or more ridges, posts, or similar guides) for properly positioning and aligning the stopping blocks 904 within the wells 912.

In embodiments of the present invention, wells of the housing or the adhesive bonding the housing to the substrate may be configured in a notched (or indented) rectangular shape, comprising two interconnected rectangular zones or chambers having such different geometries. In such indented-well configurations, the stopping block is sized to be slightly larger than the smaller rectangle but slightly smaller than the large rectangle. The benefits of this indented-well configuration are twofold. For one, it prevents the stopping block from being dragged over and damaging the cell culture during the removal process. Secondly, it minimizes the friction between the stopping block and the walls of the block-placement chamber, which, in a monolithic-well configuration comprising only a single rectangle, could be sufficiently detrimental to cause deformation of the stopping block, thereby preventing it from making conformal contact with the bottom surface of the well. The stopping block in the indented-well configuration can block the path of migrating cells without touching the edges of the well, and is therefore immune to this sort of deformation.

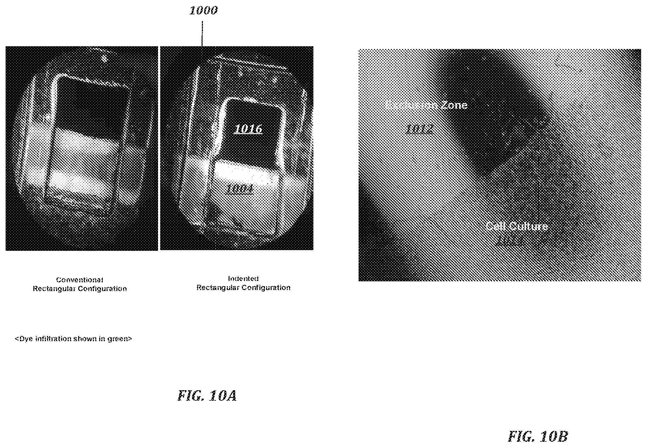

FIGURE 10A shows an exemplary embodiment of a cell-confinement structure 1000 (in this embodiment, a well 1000) that may be adapted to other embodiments of the present invention. The cell confinement structure 1000 has the indented-well configuration described above, wherein an elastomeric stopping block 1004 is inserted into a block placement chamber that would coincide with an acellular region of an underlying surface layer. FIGURE 10B illustrates a cell-exclusion zone 1012 produced according to the exemplary embodiment in FIGURE 10A, as viewed upon removal of the stopping block 1004 after 24 hours of cell seeding in a cell-seeding region 1014. In FIGURE 10A, as explained above, the rectangular block placement chamber is made larger in size than an adjoining rectangular cell seeding chamber 1016 such that the stopping block 1004 inserted within the block placement chamber achieves a better conformal seal with the walls of the entrance to the cell seeding chamber 1016 without being deformed. A leakage test performed using green dye, as shown in FIGURE 10A, demonstrates more effective fluidic confinement capabilities of the indented-well configuration, as compared to a conventional, monolithic rectangular well.

In a monolithic rectangular well format, the elastomeric stopping block being inserted between the sides of the rectangular well is likely to be deformed due to friction between itself and the sides of the well. Such deformation can result in gaps in sealing underneath the stopping blocker that allow fluid leakage therethrough. The larger size of the block-placement chamber allows the stopping block to be inserted without substantially contacting the walls of the block-placement chamber, eliminating such risk of deformation. The indented-well cell confinement structure 1000 of FIGURE 10A thus allows the stopping block 1004 to achieve a more conformal seal with the walls of the entrance to the cell seeding chamber 1016 and thus maintain a more fluid-tight seal with the cell seeding chamber 1016.

It should be noted that in any embodiment of the present invention, before or after seeding cells, the surface layer, the bottom of the wells, and/or or the substrate may be coated or covalently functionalized with molecules that influence cell behaviors such as attachment, migration, or proliferation. The cell confinement structures of the present disclosure may utilize the processes that do not contact the cell-interface surface at all (i.e., the textured first side of the surface layer), and/or that cast polymers over the cell-interface surface, or that incorporate soft substrates to cover the area into which cells will migrate. Such non-intrusive processes apply little or no force to the area they affect and thus minimally disrupt the layers of any molecules subsequently coated onto the highly textured surface layer.

Generally, after seeding the cell-seeding region of the surface layer, cells settle and attach to the cell-interface surface over time. Then, once the migration-blocking insert or other cell migration barrier is removed as described in various embodiments of the present disclosure disclosed above, the cells will begin to migrate toward the acellular region. Generally, in embodiments of the present disclosure having interdigitated electrodes, an oscillating electrical signal (either current or voltage) is applied using external hardware between the interdigitated electrodes in certain specific well or wells that were seeded with cells. The hardware may be interfaced to contact pads disposed on the top, bottom or sides of the plate, such as with spring-loaded contacts. The amplitude of the applied electrical signal may be configured to vary depending on the electrode material so that a consistent current amplitude can be maintained.

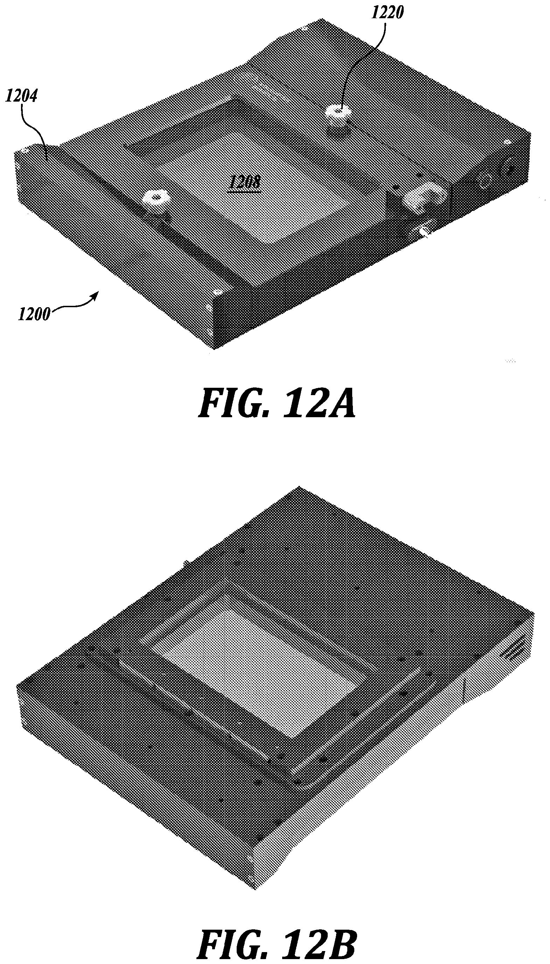

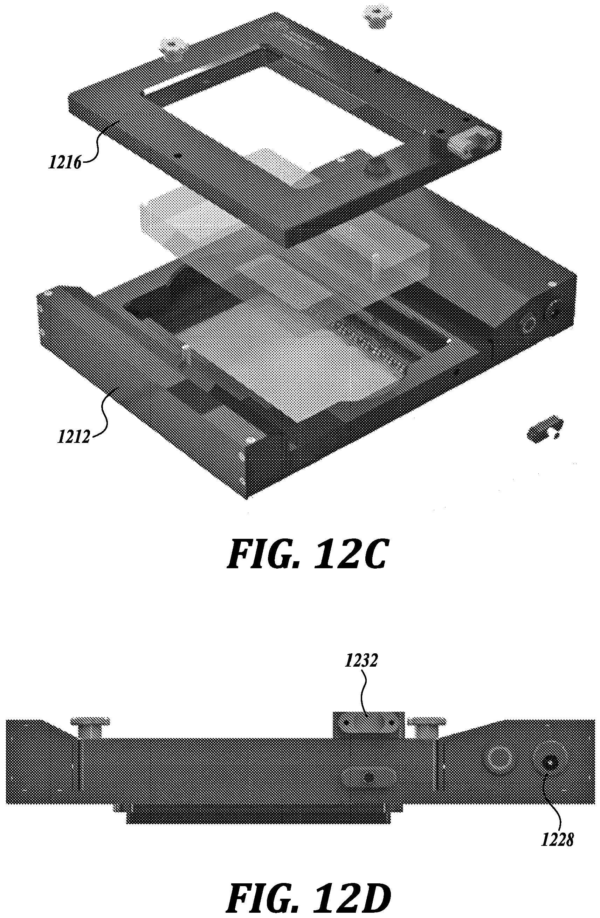

Once data on the changes in current with respect to the applied voltage (or vice-versa) are acquired (e.g., by external hardware such as the workpiece of FIGURES 12A-12D), then another set of new wells may be assayed using the same process, until all of the desired wells are analyzed. To improve throughput, in some embodiments more than one well may be analyzed simultaneously (e.g., all wells may be analyzed simultaneously) by hardware designed for such analysis (such as the workpiece of FIGURES 12A-12D). The data obtained through these processes may be used by the external hardware and/or software to calculate the impedance to the electrical current passing between the electrodes and cell culture that impede the current flows therebetween. Electrical impedance can be represented as a complex vector with a real component and an imaginary component, corresponding to the resistance and reactance of the cell culture, respectively.

From such acquired electrical data, resistance, reactance, the magnitude of the impedance vector, or the phase angle between the vector with respect to one of the complex components may be calculated and utilized for the analysis of the migratory or morphological behaviors of the cell culture.

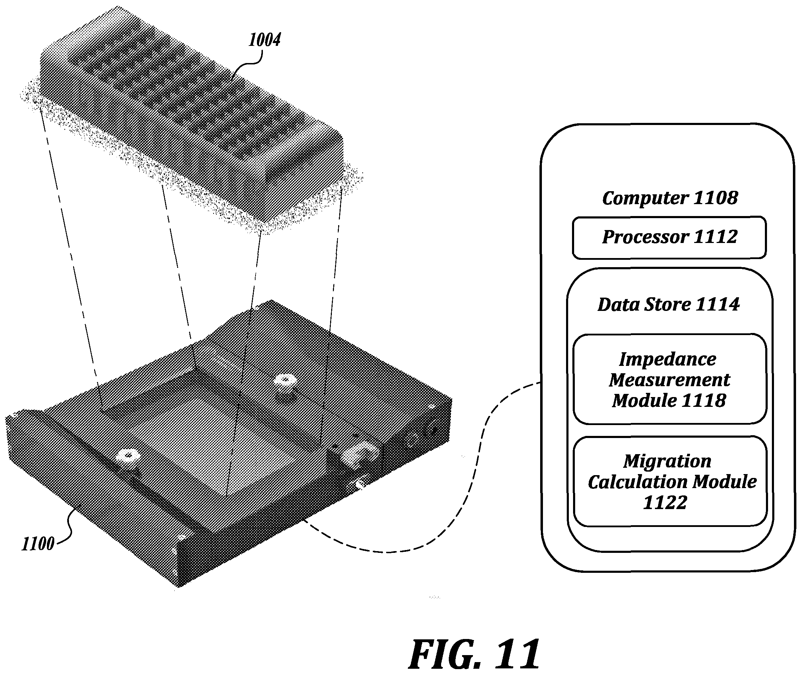

CMAPs of the present disclosure may form part of a cell analysis system comprising a workpiece. Some embodiments of the cell analysis system may include additional, separate computing resources and/or additional hardware. In the embodiment of FIGURE 11, such a cell analysis system includes a workpiece 1100 configured to securely hold a CMAP 1104 and to facilitate electrical and/or optical analysis of the cells contained therein. In particular, the workpiece 1100 is configured to operate as an impedance analyzer. Accordingly, the workpiece 1100 includes a computer 1108 having a processor 1112 (e.g., a general processing unit, a graphical processing unit, or an application specific integrated circuit); a data store 1114 (a tangible machine readable storage medium); and one or more modules that may be implemented as software logic (e.g., executable software code), firmware logic, hardware logic, or various combinations thereof. For example, the data store 1114 may include an impedance measurement module 1118 that is programmed with logic that measures the impedance between a plurality of interdigitated electrodes of the CMAP 1104. As another example, the data store 1114 may include a migration calculation module 1122 that determines a position of a cell culture on the surface layer of the CMAP 1104 and a migration distance of the cell culture across the surface layer, based upon impedance measured by the impedance measurement module 1118. The migration calculation module 1122 may optionally compute a speed of the cell migration. These functions are merely exemplary and nonlimiting. Other embodiments of the workpiece 1100 may include additional modules programmed with logic that execute any one or more of the processes described herein, or fewer modules. In some embodiments, one or more functions or structures of the computer 1108 may be implemented on a remote computer, rather than in the workpiece 1100.

FIGURES 12A-12D, illustrate one embodiment of a workpiece 1200 that may be adapted for use with any of the CMAPs of the present disclosure. The workpiece 1200 has a rigid frame 1204 that surrounds an opening 1208 sized to receive a CMAP. The frame 1204 includes a base 1212 and a removable upper frame 1216 that are together configured to securely hold a CMAP therebetween, such as with screws 1220 or similar retention devices. In an embodiment, the frame dimensions (e.g., thickness and height) may be sized to receive and hold a CMAP under or over a microscope. The workpiece 1200 is configured to transmit an electrical current, via a plurality of contact points 1224, between one or more interdigitated electrodes of a CMAP.

Consistent with FIGURE 11, the workpiece 1200 includes a processor, a data store, and one or more logic modules as described above, in order to measure the impedance between one or more interdigitated electrode pairs. The workpiece 1200 further includes a power interface 1228 to connect to a source of electrical power (e.g., a power supply) to provide the electricity for transmission across a CMAP. The workpiece 1200 further includes an optional incubation interface 1232 to connect to a heat source to facilitate cell growth within a CMAP. In some embodiments, the workpiece 1200 may include a communications interface having circuits configured to enable communication of impedance information and other information between the workpiece 1200 and a remote computer (e.g., a server, desktop computer, or laptop computer) or other network element via the internet, cellular network, RF network, Personal Area Network (PAN), Local Area Network, Wide Area Network, or other network. Accordingly, the communications interface may be configured to communicate using wireless protocols (e.g., WIFI^{®}. WIMAX^{®}, BLUETOOTH^{®}, ZIGBEE^{®}, Cellular, Infrared, Nearfield, etc.) and/or wired protocols (Universal Serial Bus or other serial communications such as RS-234, RJ-45, etc., parallel communications bus, etc.). In some embodiments, the communications interface includes circuitry configured to initiate a discovery protocol that allows the workpiece and the other network element to identify each other and exchange control information. In an embodiment, the communications interface has circuitry configured to a discovery protocol and to negotiate one or more pre-shared keys. In an embodiment, the communications interface alternatively or additional includes circuitry configured to initiate a discovery protocol that allows an enterprise server and the workpiece to exchange information.

The cell analysis systems described above, including CMAPs incorporating the interdigitated electrodes of the present disclosures are configured to allow impedance to increase substantially linearly with the migration distance of the cell monolayer. As such, the migratory behavior of the cell culture can be quantitatively evaluated and analyzed. Although correlation factors for different cell-types may differ, calibration allowing cross-comparison between different cell types can be performed in the following ways:
1. Cells in a given well or set of wells are directed to migrate to an end of an acellular region in a well. Then, the impedance values obtained from the CMAPs with all other wells can be normalized to the maximum impedance value achieved in a control well(s), and multiplied by the corresponding maximum migration distance achieved in the control wells(s) (i.e., that from the starting position in the cell-seeding region to the end of the acellular region in the well).
2. An impedance value on cells in a given well(s) can be acquired before or immediately after migration is directed to commence (e.g., by removing an insert). From this measured impedance value, the initial cell impedance can be calculated by subtracting the baseline electrode impedance. Subsequently acquired impedance values can then be normalized to the initial cell impedance and multiplied by the length of the cell-seeding region running in the direction of the cell migration.

In experiments designed to validate the proper functioning of devices, systems and methods for CMAPs of the present disclosure, MCF10A cells, a non-tumorigenic mammary epithelial cell line, were chosen as the cell model to validate whether a Nafion-nanotextured substrate is suitable for cell growth and migration. Cell monolayers were grown to cover a half of the area of each well to achieve a uniform condition at the beginning of the experiment. An elastomeric silicone (polydimethylsiloxane or PDMS) block was attached to the Nafion-patterned surface in each well, and then the MCF10A cells were seeded into the notch of the PDMS block at a seeding density of 20,000 cells per well. After 24 hours of incubation, the cells grew into a cell monolayer in the cell-seeding region and the PDMS block in each well was removed to initiate an impedimetric recording of collective cell migration. Cells migrated collectively as a cell sheet in a migration direction toward the other side of the well along the direction of nanogrooves and reached the end of the nanotextured migration area after 60-hour of migration.

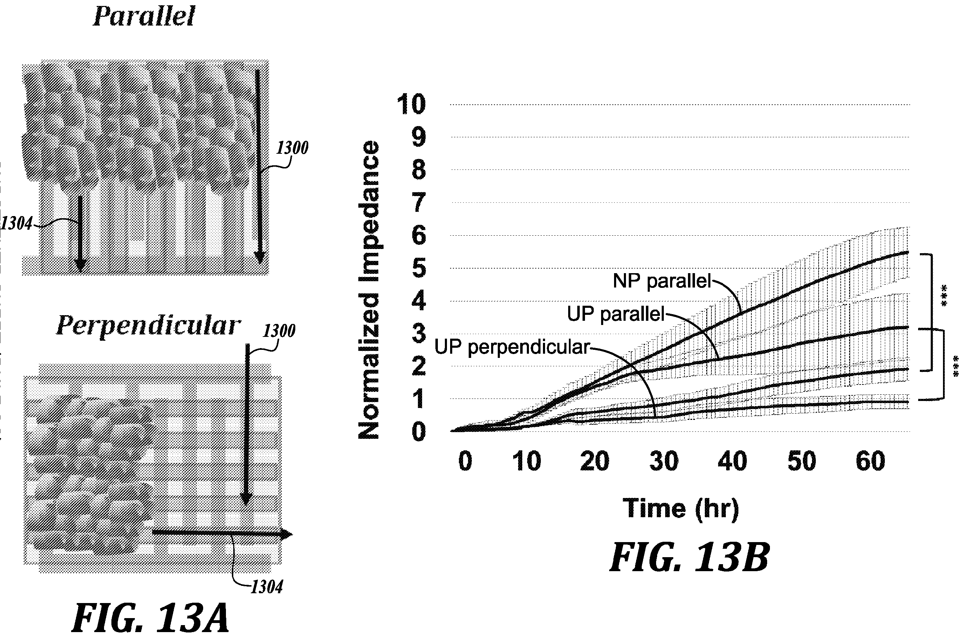

Further validation studies were carried out to determine optimal electrode configurations that can allow assessment of collective cell migration with high accuracy and sensitivity. FIGURE 13A illustrates different directional configurations of the interdigitated electrode orientation 1300 in reference to a longitudinal direction 1304 of nanopatterns formed on a cell-interfacing surface layer, wherein cells are directed to migrate either parallel or perpendicular to the electrode orientation 1300. FIGURE 13B displays normalized impedance measurements taken at 10 kHz using the nanotextured plates of the present disclosure implemented in parallel and perpendicular electrode configurations, respectively, as shown in FIGURE 13A, as compared to the same measurements obtained with both electrode configurations on a flat (or untextured) plate. All electrodes were fabricated from gold and patterned with Nafion composition as described above.

In the parallel electrode configuration, the interdigitated electrodes orientation 1300 is parallel to the longitudinal direction 1304 of the nanogrooves and cell migration. In the perpendicular electrode configuration, the electrode orienatation 1300 is perpendicular to the longitudinal direction 1304 of the nanogrooves and cell migration. The real-time migration data obtained (P < 0.001) from the validation studies in FIGURE 13B shows that, at each time point, higher impedance is observed in the parallel configuration than that of the perpendicular configuration, on both nanotextured (NP) and untextured (UP) surfaces. When the impedance values are correlated to the migration distances measured from the microscope images, no significant difference was seen between the migration distances of perpendicular and of parallel groups, but the measured impedance was significantly lower in the perpendicular group than in the parallel group (P < 0.05). The data demonstrate that differential sensitivities can be achieved with different electrode orientations. The larger difference between migration distance and impedance corresponds to reduced sensitivity of the interdigitated electrodes to cell movement. The above findings indicate that the detection ability of the interdigitated electrodes is enhanced when the digits of the interdigitated electrodes run parallel to the direction of nanogrooves and cell migration.

Additional validation studies were carried out to determine whether collective cell migration behaviors are affected by the nanotextured cell-interface surface, which mimics the topography of *in vivo* cell migration microenvironment. MCF10A cells were grown on both nanotextured and untextured surfaces and the distances between the cell sheet border at 0 hour and 60 hours were measured.

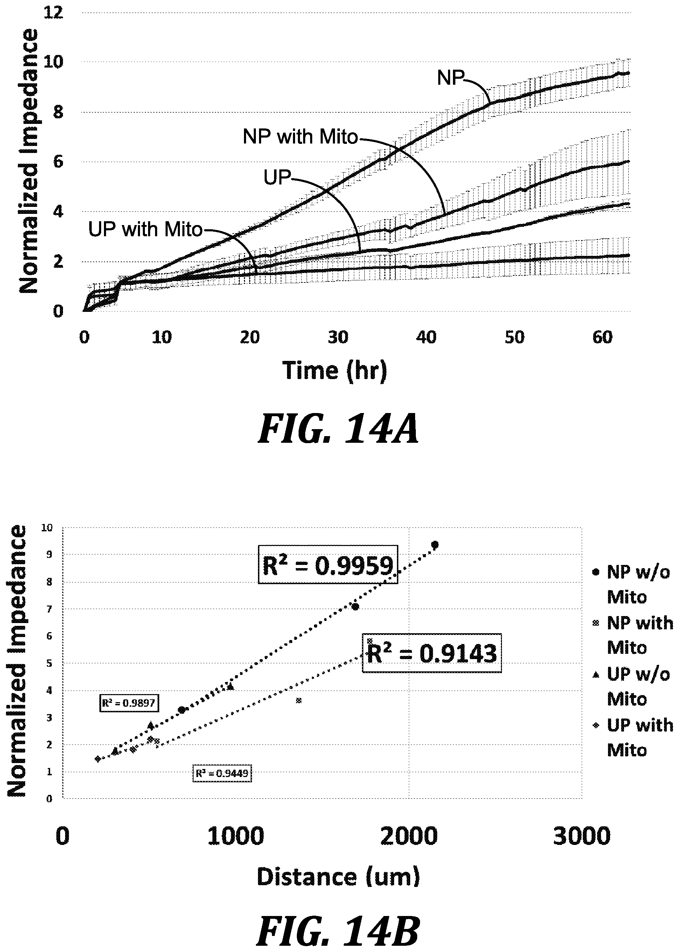

FIGURE 14A illustrates the impedimetric data taken at 10 kHz, which clearly demonstrate that nanotopography of the cell interface surface of the present disclosure has dramatic influences on cellular migration speed. The impedimetric analysis in FIGURE 14A shows that MCF10A cells had faster collective cell migration on nanotextured (NP) surfaces than on the untextured (UP) surfaces, as demonstrated by a more significant increase of impedance on NP surfaces than on UP surfaces (P < 0.001). The experimental results show that the cell culture on the nanogrooves of the present disclosure migrates twice as fast as that on the untextured surface.

Additionally, further validation studies were carried out to verify the impedimetric analysis platform of the present disclosure as a reliable real-time collective CMAP. The impedance values measured by the impedance analyzer of the present disclosure were compared and correlated with the migration distances of the collective cell layer calculated from bright field images at different time points (1st spot: 20 hours; 2nd spot: 40 hours; 3rd spot: 60 hours), as shown in FIG 14B. The data in FIGURE 14B demonstrate that the cell migration distance measured by microscopy correlates with extremely high accuracy to the impedance value measured with an exemplary device of the present disclosure. Strong positive, linear correlations between impedance and migration distance were observed within both NP (R2 = 0.9959) and UP (R2 = 0.9897) groups. Moreover, similar slopes in NP (m = 0.0041) and UP (m = 0.0035) groups further indicate a good impedance-migration distance correlation even on different surface topographies. The slight differences between these slopes could be attributable to the changes in cellular architecture in response to underlying nanotopographic cues.

Identifying effective drugs that target tumor metastasis is one of the ultimate goals of cancer migration assays. For this reason, a CMAP of the present disclosure was used to assess its capability to detect drug effects by using widely applied inhibitors. In this experiment, 1 ng/ml mitomycin C was used to inhibit MCF10A cell proliferation to determine whether collective cell migration is still faster on Nafion-nanotextured surfaces than on the untextured surfaces, after eliminating proliferation as a confounding factor. FIGURE 14A demonstrates that nanotopography of the cell interface surface of the present disclosure has dramatic influences on the resistance to mitomycin, a known cellular proliferation inhibitor. Proliferation inhibition substantially slowed but did not inhibit collective migration in both nanotextured (NP) and untextured (UP) groups, which would be expected, as collective migration is driven by cell proliferation as well as individual cell motility. Importantly, while the treated UP groups migrated minimally as expected with no significant change in impedance over the duration, the treated NP cultures continued to migrate with alacrity, even outpacing their UP untreated counterparts (P < 0.05). These data highlight the effectiveness of nanotopographic guidance in maintaining an aggressive phenotype in the face of pharmacological inhibition. Additionally, a distance-impedance correlation analysis again shows strong positive linearity within NP (R2 = 0.9143) and UP (R2 = 0.9449) groups. All of the data obtained suggest that the nanogrooved cell-interface surfaces provide a more rigorous *in vitro* screening environment than the flat surfaces in cancer drug studies.

The CMAPs of the present invention were used to further assess its capability to detect drug effects by performing a 3-dose response test with the compound, LY294002. This compound inhibits the phosphoinositide 3-kinase pathway (PI3K) on PIK3CA H1047R mutation knock-in MCF10A cells. This cell model and inhibitor was chosen because PIK3CA mutation is one of the most prevalent gene aberrations in metastatic tumors and in PI3K/Akt/mTOR pathway. After treatment with the PI3K inhibitor, both untextured (UP) and nanotextured (NP) groups showed significant dose-dependent decreases in impedance.

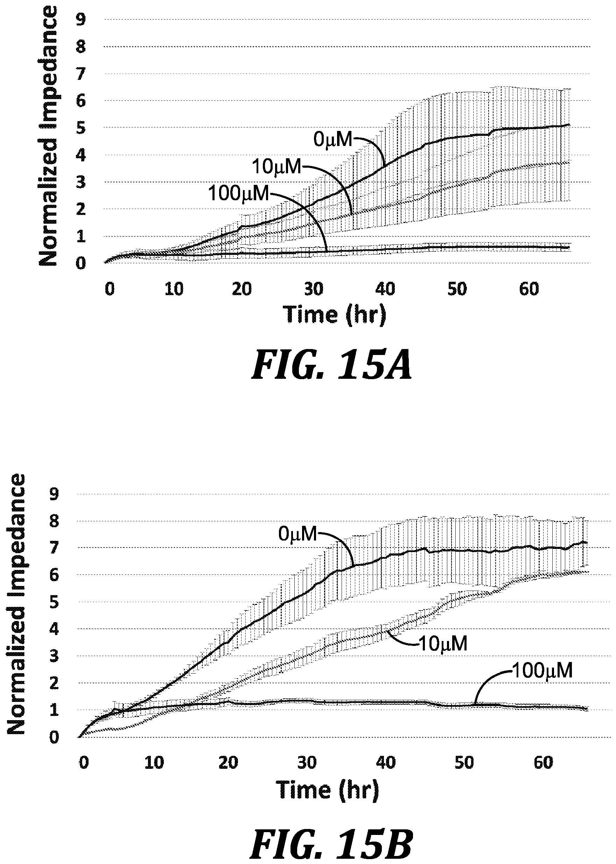

FIGURES 15A and 15B illustrate that *PIK3CA* knock-in MCF10A cells exhibit distinctly stratified responses in impedance value to different dosages of the PI3K inhibitor LY294002 on untextured (FIGURE 15A) and nanotextured (FIGURE 15B) surfaces. As drug concentrations were increased, the range of impedances measured decreased. Impedance is shown to be highly correlated to the distance traveled at low-dose treatment, compared to the minimally migrated distance at high-dose treatment, indicating that the observed results were due to migration inhibition, rather than due to the cytotoxicity of the inhibitor. Importantly, the higher stratification in migration behaviors at each dosage on nanotextured surfaces can provide resolution of substantially greater differences in migration distance than is capable on the untextured surfaces. Increased assay sensitivity can be achieved on the nanotextured cell-surface interfaces due to these differences in stratification.

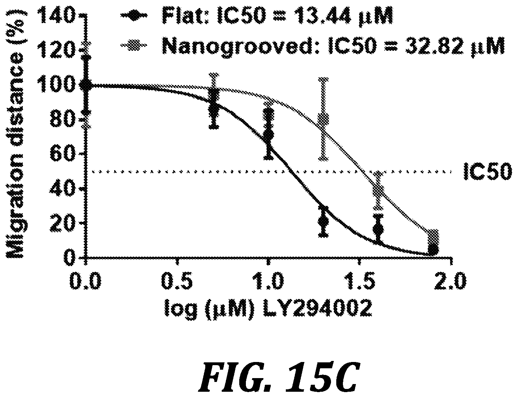

FIGURE 15C illustrates a comparative endpoint dose-response study showing enhanced resistance of the cells to the compound LY294002 when the cells are directed to migrate on a nanotextured surface vs. a flat surface. The high throughput microscopy studies reveal that higher half max inhibitory concentration (IC50) for LY294002 is higher for cells migrating on anisotropic nanopatterns. This indicates that a more drug-resistant phenotype could be provoked in migrating cells on the nanotextured surfaces of the present disclosure, by virtue of their enhanced environmental biomimicry.

In the foregoing description, specific details are set forth to provide a thorough understanding of exemplary embodiments of the present invention. It will be apparent to one skilled in the art, however, that the embodiments disclosed herein may be practiced without embodying all of the specific details. In some instances, well-known process steps have not been described in detail in order not to unnecessarily obscure various aspects of the present disclosure. Further, it will be appreciated that embodiments of the present invention may employ any combination of features described herein, within the scope of the claims.

The present application may include references to directions, such as "vertical," "horizontal," "front," "rear," "left," "right," "top," and "bottom," etc. These references, and other similar references in the present application, are only to assist in helping describe and understand the particular embodiment (such as when the embodiment is positioned for use) and are not intended to limit the present disclosure to these directions or locations.

The present application may also reference quantities and numbers. Unless specifically stated, such quantities and numbers are not to be considered restrictive, but exemplary of the possible quantities or numbers associated with the present application. Also in this regard, the present application may use the term "plurality" to reference a quantity or number. In this regard, the term "plurality" is meant to be any number that is more than one, for example, two, three, four, five, etc. The term "about," "approximately," etc., means plus or minus 5% of the stated value.

## Claims

1. An apparatus (20) for topographically guiding migration of a cell culture (48), the apparatus (20) comprising:
a substrate (36);
an ion-permeable surface layer (56) having a textured first side (72) facing away from the substrate (36), and a second side (76) that is disposed proximate to the substrate (36), the textured first side (72) having a unidirectional texture and being configurable to have a cell seeding region (84) for the cell culture (48) and an acellular region (88), wherein the unidirectional texture is configured to guide migration of the cell culture (48) toward the acellular region (88) in a substantially linear migration direction (90); and
an electrode pair (64a, 64b) comprising a first electrode (64a) and a second electrode (64b), each of the first and second electrodes (64a, 64b) having a plurality of digits (68a, 68b) disposed between the substrate (36) and the ion-permeable surface layer (56), wherein the plurality of digits (68a) of the first electrode (64a) is interdigitated with the plurality of digits (68b) of the second electrode (68b).

2. The apparatus (20) of claim 1, wherein the electrode pair (64a, 64b) and the ion-permeable surface layer (56) are configured such that an impedance to an electrical current flow between the first and second electrodes (64a, 64b) varies substantially linear in response to migration of the cell culture (48) in the substantially linear migration direction (90).

3. The apparatus (20) of claim 1, wherein the unidirectional texture comprises a plurality of substantially parallel grooves (80).

4. The apparatus (20) of claim 1, wherein the digits (68a, 68b) of the first and second electrodes (64a, 64b) are configured to run substantially parallel or substantially perpendicular to the migration direction (90).

5. The apparatus (20) of claim 1, wherein the unidirectional texture of the textured first side (72) of the ion-permeable surface layer (56) is configured to guide migration of the cell culture (48) deposited upon the cell-seeding region (84) toward the acellular region (88) in the substantially linear migration direction (90).

6. The apparatus (20) of claim 1, further comprising a cell confinement structure (32) comprising two walls (44) that are positioned proximal to the cell-seeding region (84) and to the acellular region (88), wherein the two walls (44) run substantially parallel to a longitudinal direction (52) of the unidirectional texture in order to guide migration of the cell culture (48) in the longitudinal direction (52).

7. The apparatus (20) of claim 6, wherein the cell confinement structure (32) at least partially defines a well (32) that is bounded on two sides by the two walls (44), the well (32) having a bottom area (40) in which the pluralities of digits (68a, 68b) of the first and second electrodes (64a, 64b) are disposed and in which the cell-seeding region (84) and the acellular region (88) are exposed to a cavity of the well (32).

8. The apparatus (20) of claim 7, wherein the cell confinement structure (32) further comprises an insert that can be removably inserted into the cavity of the well (32) such that it divides the cell-seeding region (84) from the acellular region (88), thereby preventing migration of the cell culture (48) from the cell-seeding region (84) into the acellular region (88).

9. The apparatus (20) of claim 8, wherein the insert is sized such that after it is removably inserted into the cavity of the well (32) and a cell suspension is added to the cell-seeding region (84), a surface tension of the cell suspension forms a barrier between the insert and the textured first side (72) of the ion-permeable surface layer (56) that prevents passage of the cell suspension through a gap formed between the insert and the textured first side (72).

10. The apparatus (20) of claim 8, wherein the insert is at least partially covered with an overmold material that is configured to expand into the cavity to further divide the cell-seeding region (84) from the acellular region (88).

11. The apparatus (20) of claim 6, wherein the cell confinement structure (32) further comprises a ceiling that spans the two walls (44), such that the two walls (44), the ceiling, and the textured first side (72) of the ion-permeable surface layer (56) define at least part of a microfluidic channel that connects the cell-seeding region (84) to the acellular region (88).

12. The apparatus (20) of claim 11, wherein the microfluidic channel further comprises a first chamber located at a first end thereof, and a second chamber located at a second end thereof, wherein the cell-seeding region (84) is located within the first chamber and the acellular region (88) is at least partially located within the second chamber.

13. The apparatus (20) of claim 1, wherein the ion-permeable surface layer (56) is a composition that includes at least one material selected from the following: Nafion, gelatin, methacrylated gelatin, peg diacrylate gels, thermoplastic track-etched membranes, polyethylene terephthalate glycol (PETG), MATRIGEL^{®}, poly-acrylamide, poly n-isopropylacrylamide (Poly-NIPAM), agarose gels, dextran gels, other crosslinked hydrogels, or other polymer electrolytes.

14. A cell analysis system, comprising:
the apparatus (20) of claim 1; and
a workpiece (1100; 1200) configured to securely hold the apparatus (20) and to transmit an electrical current between the first electrode (64a) and the second electrode (64b).

15. The cell analysis system of claim 14, wherein the workpiece (1100; 1200) is configured to measure an impedance to the electrical current transmitted by the workpiece (1100; 1200) between the first electrode (64a) and the second electrode (64b).

16. The cell analysis system of claim 15, further comprising a computer program stored on a non-transitory computer-readable medium that includes instructions that, when executed, cause a processor to perform steps, including:
determining, based on the measured impedance, a migration distance of the cell culture (48) from the cell-seeding region (84) toward the acellular region (88).

## Patentansprüche

1. Eine Vorrichtung (20) zur topographischen Führung der Migration einer Zellkultur (48), die Vorrichtung (20) umfassend:
ein Substrat (36);
eine ionendurchlässige Oberflächenschicht (56) mit einer texturierten ersten Seite (72), die von dem Substrat (36) abgewandt ist, und einer zweiten Seite (76), die in der Nähe des Substrats (36) angeordnet ist, wobei die texturierte erste Seite (72) eine unidirektionale Textur aufweist und so konfigurierbar ist, dass sie einen Zellaussaatbereich (84) für die Zellkultur (48) und einen azellulären Bereich (88) aufweist, wobei die unidirektionale Textur so konfiguriert ist, dass sie die Migration der Zellkultur (48) in Richtung des azellulären Bereichs (88) in einer im Wesentlichen linearen Migrationsrichtung (90) leitet; und
ein Elektrodenpaar (64a, 64b), das eine erste Elektrode (64a) und eine zweite Elektrode (64b) umfasst, wobei jede der ersten und zweiten Elektrode (64a, 64b) eine Vielzahl von Stellen (68a, 68b) aufweist, die zwischen dem Substrat (36) und der ionendurchlässigen Oberflächenschicht (56) angeordnet sind, wobei die Vielzahl von Stellen (68a) der ersten Elektrode (64a) mit der Vielzahl von Stellen (68b) der zweiten Elektrode (68b) verflochten ist.

2. Die Vorrichtung (20) nach Anspruch 1, wobei das Elektrodenpaar (64a, 64b) und die ionendurchlässige Oberflächenschicht (56) so konfiguriert sind, dass eine Impedanz für einen elektrischen Stromfluss zwischen der ersten und der zweiten Elektrode (64a, 64b) in Reaktion auf die Migration der Zellkultur (48) in der im Wesentlichen linearen Migrationsrichtung (90) im Wesentlichen linear variiert.

3. Die Vorrichtung (20) nach Anspruch 1, wobei die unidirektionale Textur eine Vielzahl von im Wesentlichen parallelen Rillen (80) umfasst.

4. Die Vorrichtung (20) nach Anspruch 1, wobei die Stellen (68a, 68b) der ersten und zweiten Elektrode (64a, 64b) so gestaltet sind, dass sie im Wesentlichen parallel oder im Wesentlichen senkrecht zur Migrationsrichtung (90) verlaufen.

5. Die Vorrichtung (20) nach Anspruch 1, wobei die unidirektionale Textur der texturierten ersten Seite (72) der ionendurchlässigen Oberflächenschicht (56) so konfiguriert ist, dass sie die Migration der auf dem Zellaussaatbereich (84) abgelagerten Zellkultur (48) zu dem azellulären Bereich (88) in der im Wesentlichen linearen Migrationsrichtung (90) leitet.

6. Die Vorrichtung (20) nach Anspruch 1, die ferner eine Zellbegrenzungsstruktur (32) umfasst, die zwei Wände (44) umfasst, die in der Nähe des Zellaussaatbereichs (84) und des azellulären Bereichs (88) angeordnet sind, wobei die beiden Wände (44) im Wesentlichen parallel zu einer Längsrichtung (52) der unidirektionalen Textur verlaufen, um die Migration der Zellkultur (48) in der Längsrichtung (52) zu lenken.

7. Die Vorrichtung (20) nach Anspruch 6, wobei die Zellbegrenzungsstruktur (32) zumindest teilweise eine Vertiefung (32) definiert, die an zwei Seiten durch die beiden Wände (44) begrenzt ist, wobei die Vertiefung (32) einen Bodenbereich (40) aufweist, in dem die Mehrzahl von Stellen (68a, 68b) der ersten und zweiten Elektroden (64a, 64b) angeordnet sind und in dem der Zellaussaatbereich (84) und der azelluläre Bereich (88) einem Hohlraum der Vertiefung (32) ausgesetzt sind.

8. Die Vorrichtung (20) nach Anspruch 7, wobei die Zellbegrenzungsstruktur (32) ferner einen Einsatz umfasst, der herausnehmbar in den Hohlraum der Vertiefung (32) eingesetzt werden kann, so, dass er den Zellaussaatbereich (84) von dem azellulären Bereich (88) trennt, wodurch die Migration der Zellkultur (48) aus dem Zellaussaatbereich (84) in den azellulären Bereich (88) verhindert wird.

9. Die Vorrichtung (20) nach Anspruch 8, wobei der Einsatz so bemessen ist, dass, nachdem er herausnehmbar in den Hohlraum der Vertiefung (32) eingesetzt und eine Zellsuspension in den Zellaussaatbereich (84) gegeben wurde, eine Oberflächenspannung der Zellsuspension eine Barriere zwischen dem Einsatz und der texturierten ersten Seite (72) der ionendurchlässigen Oberflächenschicht (56) bildet, die den Durchgang der Zellsuspension durch einen zwischen dem Einsatz und der texturierten ersten Seite (72) gebildeten Spalt verhindert.

10. Die Vorrichtung (20) nach Anspruch 8, wobei der Einsatz zumindest teilweise mit einem Overmold-Material bedeckt ist, das so konfiguriert ist, dass es sich in den Hohlraum ausdehnt, um den Zellaussaatbereich (84) weiter von dem azellulären Bereich (88) zu trennen.

11. Die Vorrichtung (20) nach Anspruch 6, wobei die Zellbegrenzungsstruktur (32) ferner eine Decke umfasst, die die beiden Wände (44) überspannt, so dass die beiden Wände (44), die Decke und die texturierte erste Seite (72) der ionendurchlässigen Oberflächenschicht (56) zumindest einen Teil eines mikrofluidischen Kanals definieren, der den Zellaussaatbereich (84) mit dem azellulären Bereich (88) verbindet.

12. Die Vorrichtung (20) nach Anspruch 11, wobei der mikrofluidische Kanal ferner eine erste Kammer, die sich an einem ersten Ende desselben befindet, und eine zweite Kammer, die sich an einem zweiten Ende desselben befindet, umfasst, wobei sich der Zellaussaatbereich (84) innerhalb der ersten Kammer befindet und der azelluläre Bereich (88) sich zumindest teilweise innerhalb der zweiten Kammer befindet.

13. Die Vorrichtung (20) nach Anspruch 1, wobei die ionendurchlässige Oberflächenschicht (56) eine Zusammensetzung ist, die mindestens ein Material enthält, das aus den folgenden ausgewählt ist: Nafion, Gelatine, methacrylierte Gelatine, Peg-Diacrylat-Gele, thermoplastische spurgeätzte Membranen, Polyethylenterephthalatglykol (PETG), MATRIGEL^{®}, Polyacrylamid, Poly-n-isopropylacrylamid (Poly-NIPAM), Agarosegele, Dextrangele, andere vernetzte Hydrogele oder andere Polymerelektrolyte.

14. Ein Zellanalysesystem, umfassend:
die Vorrichtung (20) nach Anspruch 1; und
ein Werkstück (1100; 1200), das so konfiguriert ist, dass es die Vorrichtung (20) sicher hält und einen elektrischen Strom zwischen der ersten Elektrode (64a) und der zweiten Elektrode (64b) überträgt.

15. Das Zellanalysesystem nach Anspruch 14, wobei das Werkstück (1100; 1200) so konfiguriert ist, dass es eine Impedanz für den elektrischen Strom misst, der von dem Werkstück (1100; 1200) zwischen der ersten Elektrode (64a) und der zweiten Elektrode (64b) übertragen wird.

16. Das Zellanalysesystem nach Anspruch 15, ferner ein Computerprogramm umfassend, das auf einem nicht-transitorischen computerlesbaren Medium gespeichert ist, das Anweisungen enthält, die, wenn sie ausgeführt werden, einen Prozessor veranlassen, Schritte auszuführen, einschließlich:
Bestimmen, basierend auf der gemessenen Impedanz, einer Migrationsdistanz der Zellkultur (48) von dem Zellaussaatbereich (84) in Richtung des azellulären Bereichs (88).

## Revendications

1. Appareil (20) pour guider topographiquement la migration d'une culture cellulaire (48), l'appareil (20) comprenant :
un substrat (36) ;
une couche superficielle perméable aux ions (56) ayant un premier côté texturé (72) opposé au substrat (36), et un deuxième côté (76) qui est disposé à proximité du substrat (36), le premier côté texturé (72) ayant une texture unidirectionnelle et étant configurable pour avoir une région d'ensemencement cellulaire (84) pour la culture cellulaire (48) et une région acellulaire (88), où la texture unidirectionnelle est configurée pour guider la migration de la culture cellulaire (48) vers le région acellulaire (88) dans une direction de migration sensiblement linéaire (90) ; et
une paire d'électrodes (64a, 64b) comprenant une première électrode (64a) et une deuxième électrode (64b), chacune des première et deuxième électrodes (64a, 64b) ayant une pluralité de doigts (68a, 68b) disposés entre le substrat (36) et la couche superficielle perméable aux ions (56), où la pluralité de doigts (68a) de la première électrode (64a) est interdigitée avec la pluralité de doigts (68b) de la deuxième électrode (68b).

2. Appareil (20) de la revendication 1, dans lequel la paire d'électrodes (64a, 64b) et la couche superficielle perméable aux ions (56) sont configurées de sorte qu'une impédance à un courant électrique circulant entre les première et deuxième électrodes (64a, 64b) varie de manière sensiblement linéaire en réponse à la migration de la culture cellulaire (48) dans la direction de migration sensiblement linéaire (90).

3. Appareil (20) de la revendication 1, dans lequel la texture unidirectionnelle comprend une pluralité de rainures sensiblement parallèles (80).

4. Appareil (20) de la revendication 1, dans lequel les doigts (68a, 68b) des première et deuxième électrodes (64a, 64b) sont configurés pour s'étendre de manière sensiblement parallèle ou de manière sensiblement perpendiculaire à la direction de migration (90).

5. Appareil (20) de la revendication 1, dans lequel la texture unidirectionnelle du premier côté texturé (72) de la couche superficielle perméable aux ions (56) est configurée pour guider la migration de la culture cellulaire (48) déposée sur la région d'ensemencement cellulaire (84) vers la région acellulaire (88) dans la direction de migration sensiblement linéaire (90).

6. Appareil (20) de la revendication 1, comprenant en outre une structure de confinement cellulaire (32) comprenant deux parois (44) qui sont positionnées à proximité de la région d'ensemencement cellulaire (84) et de la région acellulaire (88), où les deux parois (44) s'étendent de manière sensiblement parallèle à une direction longitudinale (52) de la texture unidirectionnelle afin de guider la migration de la culture cellulaire (48) dans la direction longitudinale (52).

7. Appareil (20) de la revendication 6, dans lequel la structure de confinement cellulaire (32) définit au moins partiellement un puits (32) qui est délimité sur deux côtés par les deux parois (44), le puits (32) ayant une zone de fond (40) dans laquelle sont disposées les pluralités de doigts (68a, 68b) des première et deuxième électrodes (64a, 64b) et dans laquelle la région d'ensemencement cellulaire (84) et la région acellulaire (88) sont exposées à une cavité du puits (32).

8. Appareil (20) de la revendication 7, dans lequel la structure de confinement cellulaire (32) comprend en outre un insert qui peut être inséré de manière amovible dans la cavité du puits (82) de sorte qu'il divise la région d'ensemencement cellulaire (84) de la région acellulaire (88), empêchant ainsi la migration de la culture cellulaire (48) de la région d'ensemencement cellulaire (84) vers la région acellulaire (88).

9. Appareil (20) de la revendication 8, dans lequel l'insert est dimensionné de sorte que, après avoir été inséré de manière amovible dans la cavité du puits (32) et qu'une suspension cellulaire a été ajoutée à la région d'ensemencement cellulaire (84), une tension superficielle de la suspension cellulaire forme une barrière entre l'insert et le premier côté texturé (72) de la couche superficielle perméable aux ions (56) qui empêche le passage de la suspension cellulaire à travers un espace formé entre l'insert et le premier côté texturé (72).

10. Appareil (20) de la revendication 8, dans lequel l'insert est au moins partiellement recouvert d'un matériau de surmoulage qui est configuré pour se dilater dans la cavité pour diviser davantage la région d'ensemencement cellulaire (84) de la région acellulaire (88).

11. Appareil (20) de la revendication 6, dans lequel la structure de confinement cellulaire (32) comprend en outre un plafond qui enjambe les deux parois (44), de sorte que les deux parois (44), le plafond et le premier côté texturé (72) de la couche superficielle perméable aux ions (56) définissent au moins une partie d'un canal microfluidique qui relie la région d'ensemencement cellulaire (84) à la région acellulaire (88).

12. Appareil (20) de la revendication 11, dans lequel le canal microfluidique comprend en outre une première chambre située au niveau d'une première extrémité de celui-ci, et une deuxième chambre située au niveau d'une deuxième extrémité de celui-ci, où la région d'ensemencement cellulaire (84) est située à l'intérieur de la première chambre et la région acellulaire (88) est au moins partiellement située à l'intérieur de la deuxième chambre.

13. Appareil (20) de la revendication 1, dans lequel la couche superficielle perméable aux ions (56) est une composition qui comprend au moins un matériau choisi parmi les matériaux suivants : Nafion, gélatine, gélatine méthacrylée, gels de peg-diacrylate, membranes thermoplastiques à traces attaquées, polyéthylène téréphtalate glycol (PETG), MATRIGEL^{®}, poly-acrylamide, poly n-isopropylacrylamide (Poly-NIPAM), gels d'agarose, gels de dextrane, autres hydrogels réticulés ou autres électrolytes polymères.

14. Système d'analyse cellulaire, comprenant :
l'appareil (20) de la revendication 1 ; et
une pièce à usiner (1100 ; 1200) configurée pour maintenir solidement l'appareil (20) et pour transmettre un courant électrique entre la première électrode (64a) et la deuxième électrode (64b).

15. Système d'analyse cellulaire de la revendication 14, dans lequel la pièce à usiner (1100 ; 1200) est configurée pour mesurer une impédance au courant électrique transmis par la pièce à usiner (1100 ; 1200) entre la première électrode (64a) et la deuxième électrode (64b).

16. Système d'analyse cellulaire de la revendication 15, comprenant en outre un programme informatique stocké sur un support non transitoire lisible par ordinateur qui comprend des instructions qui, lorsqu'elles sont exécutées, amènent un processeur à effectuer des étapes, comprenant :
déterminer, sur la base de l'impédance mesurée, une distance de migration de la culture cellulaire (48) de la région d'ensemencement cellulaire (84) vers la région acellulaire (88).
